# EUROPEAN PATENT APPLICATION

(11) **EP 2 466 310 A1**
(43) Date of publication of application: **20.06.2012**
(21) Application number: 10195555.7
(22) Date of filing: 17.12.2010
(51) Int. Cl.: G01N 33/68

(54) **Means and methods for detecting double-strand breaks**

(71) Applicant: Helmholtz Zentrum München Deutsches Forschungszentrum für Gesundheit und Umwelt GmbH, 85764 Neuherberg (DE)
(72) Inventor: The designation of the inventor has not yet been filed
(74) Representative: Schiweck, Weinzierl & Koch

(57) **Abstract**

The present invention makes use of proximity ligation in a method for determining the level of DNA damage, in particular, in cells of a subject subjected to ionizing radiation, which is based on the quantification of DNA repair proteins congregating at sites of DNA repair. Accordingly, the present invention provides a new approach to monitor radiation exposure by the detection of DNA double strand breaks. Specifically, the methods of the present invention are capable to visualize a linear dose response relationship in the biologically relevant range of 0.2 to 2 Gy by translating DNA damages into detectable signals by way of the application of a proximity ligation assay.

## Description

### TECHNICAL FIELD

The present invention makes use of proximity ligation in a method for determining the level of DNA damage, in particular, in cells of a subject subjected to ionizing radiation, which is based on the quantification of DNA repair proteins congregating at sites of DNA repair. Accordingly, the present invention provides a new approach to monitor radiation exposure by the detection of DNA double strand breaks. Specifically, the methods of the present invention are capable to visualize a linear dose response relationship in the biologically relevant range of 0.2 to 2 Gy by translating DNA damages into detectable signals by way of the application of a proximity ligation assay.

### BACKGROUND OF THE INVENTION

DNA damage and cell killing result from the effects of ionizing radiation on the human and animal body. In many examples, the effects are proportional to the dose rate. Ionizing radiation has been postulated to induce multiple biological effects by direct interaction with DNA or through the formation of free radical species leading to DNA damage. These effects include gene mutations, malignant transformation, and cell killing.

Exposure to ionizing radiation (IR) induces a range of damages, including single strand breaks, double strand breaks (DSB), base damage and DNA-protein cross-links in the genomic DNA. Among them, DSBs are the most deleterious lesion, which potentially lead to genomic instability and cell death when they are mis- or unrepaired. Exposure to ionizing radiation triggers a large-scale activation of specific DNA damage signaling and repair mechanisms. This contains the phosphorylation of H2AX over large chromatin domains surrounding the DSB [1, 2, 3]. Phosphorylation of H2AX is mediated by the phosphoinositide 3-kinase-related kinase (PIKK) family members ATM and DNA-PK after ionizing radiation [4] whereas ATR and, to a lesser extent, DNA-PK appear to be involved in γ-H2AX formation at sites of replication-associated breaks [5]. Foci yields have been shown to increase linearly with dose and the initial number of γ-H2AX foci formed per cell nucleus following IR coincides with the yield of induced DSBs [6]. Foci disappearance over time reflects DSB repair. The two major DSB repair pathways in eukaryotic cells are non-homologous end joining (NHEJ) and homologous recombination (HR) repair [7]. Some important players in NHEJ include DNA ligase IV/XRCC4 (X-ray cross complementation group 4) DNA-dependent protein kinase (DNA-PK), Ku70/Ku80 and Artemis. In HR, RAD51 protein and its paralogues RAD52 and RAD54 have important roles. Additionally, several accessory proteins designated as damage sensors and mediators, signal transducers and effectors participate in DNA damage signaling and are involved in DSB repair (*e.g*., BRCA1, 53BP1, MDC1, CHk1/2, Mre11, Nbs1, RAD50).

Thus far, biological dosimetry of radiation exposure is mainly insensitive, requires specialized staff, has limited capacity, and, importantly, available assays are non-linear and take 3 days to perform using conventional approaches such as pulsed-field electrophoresis, karyograms and the like. This makes these approaches, more or less, useless for rapid quantification of exposure in situations where an immediate read-out is necessary such as a nuclear emergency (e.g., nuclear accidents, nuclear incidents) or during radiotherapy (clinical control).

Currently, foci scoring of DNA damage processing proteins by fluorescence microscopy (especially monitoring of gamma-H2AX) appears to be a valuable tool for the analysis of DNA double strand break processing. However, the main disadvantages of foci scoring are the highly dynamic change in foci numbers, size and intensity which makes the scoring process somewhat difficult and thus not applicable for a rapid and reliable, preferably a mostly automatable assay which enables a linear readout of DNA damage.

Accordingly, given the drawbacks of the thus-far available means and methods for detection, visualization and, in particular, quantification of DNA damage, there is a need to provide rapid, robust and sensitive means and methods for the detection, visualization and/or quantification of DNA damage in cells, particularly in cells of a subject.

Thus, the technical problem of the present invention is to comply with this need.

### SUMMARY OF THE INVENTION

The present inventors used the proximity ligation assay (PLA) to quantify the level of DNA damage in cells subjected to ionizing radiation. Commonly, the proximity ligation assay is applied to detect and quantify protein interaction (e.g., homodimeric complexes or high-throughput for screening potential protein interactions), protein phosphorylation or protein expression. However, unless the interaction of two proteins is a priori known or assumed, the PLA technology has so far not been described to be applicable for unknown or not yet identified protein interactions. Moreover, a diagnostic application of the proximity ligation assay has thus far neither been described nor suggested.

The assay as applied by the present inventors implemented their profound knowledge of DNA repair mechanisms into the context of a possible application of the proximity ligation assay. Specifically, the assay as applied by the inventors uses binding moieties such as antibodies, preferably monoclonal antibodies, to bind to physically adjacent epitopes on the same or different protein(s) which is/are present in a double DSB repair complex. Indeed, the most prominent damage caused by ionizing radiation is double strand breaks. The (primary) binding moieties bind to the protein to be detected. Secondary binding moieties conjugated with oligonucleotides (PLA probe 1 and PLA probe 2) are added. Upon binding of the secondary binding moieties to the primary binding moieties, the secondary binding moieties are used to drive a DNA-based amplification such as rolling circle replication. More specifically, the complementary PLA probes 1 and 2 will hybridize and thus nucleic acid amplification can be initiated, thereby allowing to detect and/or quantify the presence and/or interaction of proteins. Accordingly, DSBs can thus be localized, detected and quantified.

To their surprise, the present inventors found that the application of the proximity ligation asay for determining the level of DNA damage by numerical quantification of DNA damage in (irradiated) cells, in particular, of a subject subjected to ionizing radiation gave a high sensitivity (and low background).
Moreover, they found that the proximity ligation assay enables a linear relationship between does and detected foci (place of DSB repair) and that the sensitivity of the assay is such that a dose of down to 0.2 Gy is detectable. This value is well below thresholds of current cytogenetic-based assays for determining DNA damage in radiation exposed subjects.
In addition, they found that their assay detects subjects with genetic defects influencing DNA repair, thereby allowing a correct doses in radiotherapy.
The assay established by the present inventors is scalable and can thus deal with mass casuality situations where thousands of subjects may require screening (nucelar incident). The assay is therefore adaptable to high-throughput screening and such screening can be automated by using, for example, fluorescent labelling coupled to a plate reader.

In sum, the inventors of the present application have developed a method for determining the level of DNA damage in cells, preferably of a subject, subjected to ionizing radiation, which is based on quantifying DNA repair proteins congregating at sites of DNA repair. Accordingly, the present invention provides a new approach to monitor radiation exposure by the detection of DNA double strand breaks. This is done by visualizing the interaction between DNA damage response proteins using a modified proximity ligation method, thereby the present inventors achieved a linear dose response relationship in the biologically relevant range of up to 2 Gy, and have shown that discrimination between 0.2 and 2.0 Gy is possible. These features make the assay developed by the present inventors a valuable tool in various applications concerning (potential) DNA damage such as radiotherapy or mass screening of subjects after a nuclear incident.

Aspects of the invention are:
(1) A method for determining the level of DNA damage in cells of a subject subjected to ionizing radiation comprising (a) contacting a sample comprising cells obtained from said subject with at least two antibodies (or aptamers or anticalins) which bind to epitopes on the same or different protein(s) which is/are present in DNA repair foci; (b) contacting said sample with at least a first and second proximity probes each comprising a binding moiety with affinity for said antibodies and a nucleic acid acting as a reactive functionality, coupled thereto; (c) allowing the binding moiety of said proximity probes to bind to said antibodies and allowing the nucleic acids of said proximity probes to interact with each other if the proximity probes are in close proximity to each other; and (d) detecting the degree of interaction between the nucleic acids, thereby determining the level of DNA damage.
(2) The method according to item (1), further comprising amplifiying the interacted nucleic acids.
(3) The method of item (1) or (2), wherein determining the level of DNA damage is qualitatively and/or quantitatively.
(4) The method of any one of the preceding items, wherein the interaction between the nucleic acids in step (d) generates a detectable signal.
(5) The method of item (4), wherein the detectable signal is fluorescence, bioluminescence or radiation.
(6) The method of item (5), wherein said detectable signal is linearly related to the radiation dose to which cells of a subject were subjected.
(7) The method of item (6), wherein the linearity is given within a radiation dose range from about 0.1 Gy to about 6.0 Gy, preferably within a radiation dose range from about 0.2 Gy to about 2.0 Gy.
(8) The method of any one of the preceding items, wherein the binding moiety of the proximity probe is an antibody or aptamer.
(9) The method of item (8), wherein said antibody is directed against the Fc portion of the antibodies which bind to protein(s) which is/are present in DNA repair foci.
(10) The method of item (1), (8) or (9), wherein the antibody is monoclonal or polyclonal.
(11) The method of any one of the preceding items, wherein the DNA damage comprises double strand breaks (DSB).
(12) The method of any one of the preceding items, wherein the protein(s) which is/are present in DNA repair foci is/are DNA repair protein(s).
(13) The method of item (12), wherein said DNA repair protein is selected from the group consisting of H2AX, γH2AX, 53BP1, TRF2, H2AX, ERK, RAD50, RAD51, RAD54, Ku, XRCC4, NBS1, MRE11, MDC1, ATM, Ku70, Ku80, DNA-PK, Ligase-4, XRCC4. WRN, BLM, RPA, RNF168, BRCA1, RNF8 and Rap80.
(14) The method of any one of the preceding items, wherein the interaction of said nucleic acids coupled to the binding moieties is through hybridization to a common splint template and ligation of the nucleic acid ends.
(15) The method of any one of the preceding items, comprising (b') contacting in step (b) said sample with a third proximity probe which comprises a binding moiety with affinity for an antibody which binds to an epitope on the same or different protein(s) which is/are present in DNA repair foci, and a third nucleic acid acting as a reactive functionality, coupled thereto, the nucleic acid of said first proximity probe has a 3' free nucleic acid (A), the nucleic acid of said second proximity probe has a 5' free nucleic acid (B), and the nucleic acid of said third proximity probe has both 3' and 5' free nucleic acids (C), wherein the 3'-end of A interacts with the 5'-end of C and the 3'-end of C interacts with the 5'-end of B; and (c') conjugating the nucleic acids of said first and second proximity probes.
(16) The method of item (15), wherein the nucleic acid of said third proximity probe is a splint which is capable of hybridizing at least to the nucleic acid of said first and second proximity probes, wherein when all of the three proximity probes bind to said antibodies, the nucleic acid of said first and second proximity probes are directly or indirectly conjugatable (or conjugated) by means of a ligation reaction templated by said hybridized splint of said third proximity probe.
(17) The method of item (16), wherein the nucleic acid of said first and second proximity probes hybridize, immediately adjacent to each other to the nucleic acid of said third proximity probe.
(18) The method of item (16) or (17), wherein the nucleic acid of said first and second proximity probes hybridize, not immediately adjacent to each other, to the nucleic acid of said third proximity probe, such that when hybridized to the nucleic acid domain of said third proximity probe the nucleic acid of said first and second proximity probes are separated by a gap.
(19) The method of item (18), wherein a cassette oligonucleotide hybridizes to the nucleic acid of said third proximity probe in the gap between the respective ends of the nucleic acid of said first and second proximity probes hybridized to the nucleic acid of said third proximity probe.
(20) The method of item (18) or (19), wherein conjugation of the nucleic acid of said first and second proximity probes occurs indirectly through the cassette oligonucleotide.
(21) The method of item (18), wherein said gap between the nucleic acid of said first and second proximity probes is filled by enzymatic extension of the end of one of said first and second proximity probe nucleic acid.
(22) The method of any one of the preceding items, wherein single strand binding protein is present in the reaction mixture.
(23) The method of any one of the preceding items, wherein blocking oligonucleotides capable of binding to the free ends of the nucleic acid of said first and second proximity probes are present in the reaction mixture.
(24) The method of any one of the preceding items, wherein said blocking oligonucleotides are pre-incubated with at least said first and second proximity probes.
(25) The method of any one of the preceding items, wherein at least one of said at least first, second and third proximity probes is immobilized, or is capable of being immobilized, on a solid phase.
(26) The method of any one of the preceding items, wherein the step of detecting the interaction includes detecting the conjugation product.
(27) The method of item (26), wherein detecting the conjugation product comprises amplifying the conjugation product and detecting the amplified product.
(28) The method of any one of the preceding items, comprising multiplex analysis using several pairs of proximity probes each with a specific binding moiety and unique nucleic acids for identification.
(29) The method of any one of the preceding items, wherein said cells are human cells.
(30) The method of item (29), wherein said cells are peripheral blood mononuclear cells (PBMC) or lymphoblastoid cells.
(31) Use of the method of any one of the preceding items for (i) detecting subjects which are hypersensitive to radiation therapy; (ii) avoiding radiation injury to subjects before receiving further radiation doses; (iii) stratifying subjects in need of a countermeasure against radiation injury; or (iv) fast detection of subjects exposed to radiation.
(32) Use of the proximity ligation assay (as described in EP-B1 1 255 861 and EP-B1 1 996 941) and at least two antibodies (or aptamers or anticalins or a further binding moiety as described herein) for determining the level of DNA damage in cells of a subject subjected to ionizing radiation.
(33) A kit for determining the level of DNA damage in cells of a subject subjected to ionizing radiation comprising at least one set of at least first and second proximity probes as defined in item (1).
(34) The kit of item (33) further comprising a third proximity probe as defined in item (15) or (16).
(35) The kit of item (33) or (34), further comprising: (a) means for conjugating the nucleic acid of said first and second proximity probes; and/or (b) means for detecting said conjugation.
(36) The kit of item (35), wherein said means for conjugating the nucleic acid of said first and second proximity probes comprises a nucleic acid ligase.
(37) The kit of item (35), wherein said means for detecting said conjugation is a label or means for amplification and/or the detection thereof.
(38) The kit of any one of items (33) to (37) further comprising one or more of: (a) cassette oligonucleotide; (b) blocking oligonucleotides for the nucleic acid of said first and second proximity probes; and (c) single strand binding protein.
(39) A device suitable for determining the level of DNA damage in cells of a subject subjected to ionizing radiation in accordance with the method of any one of the afore mentioned items.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.
In each instance herein any of the terms "comprising", "consisting essentially of" and "consisting of" may be replaced with either of the other two terms.

As described herein, "preferred embodiment" means "preferred embodiment of the present invention". Likewise, as described herein, "various embodiments" and "another embodiment" means "various embodiments of the present invention" and "another embodiment of the present invention", respectively.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

### DETAILED DESCRIPTION OF THE INVENTION

Dosimetric evaluation of individuals exposed to ionizing radiation continues to pose a problem, in particular for emergency management. In mass casualty situations high throughput biological dosimetry is needed for triage. However, also in other situations like clinical situations of cancer radiotherapy of a cancer patient an emergency need may arise for dosimetric evaluation of individuals exposed to ionizing radiation. Though cytogenetic methods are available, they are time consuming and thus too slow and ineffective in order to satisfy the needs of the above mentioned scenarios. Moreover, highly skilled personnel and facilities are required for these cytogenetic methods, however such personnel is not widely and rapidly available.

Immunohistochemical quantification of DNA repair foci is an alternative method, with the advantages of robustness, scalability and speed of turnover. The current standard for DNA foci analysis remains the immunohistochemical detection of gamma-H2AX protein (phosphorylated H2AX protein). Yet, this technique suffers from disadvantages, since foci scoring undergoes highly dynamic changes in foci numbers, size and intensity which makes the scoring process somewhat difficult and thus not applicable for a rapid and reliable, preferably a mostly automatabale assay which enables a linear readout of DNA damage

The inventors of the present application have developed a method for determining the level of DNA damage in cells of a subject subjected to ionizing radiation which is based on the detection of the DNA damage response proteins at sites of DNA damage and repair. The method shows an improvement in sensitivity over known methods by reducing background signal.

The term "ionizing radiation" is well known to the person skilled in the art and consists of subatomic particles or electromagnetic waves energetic enough to detach electrons from atoms or molecules, thus ionizing them. The degree and nature of such ionization depends on the energy of the individual particles or waves, and not on their number. An intense flood of particles or waves will not cause ionization if these particles or waves do not carry enough energy to be ionizing. Roughly speaking, particles or photons with energies above a few electron volts (eV) are ionizing. Examples of ionizing particles are energetic alpha particles, beta particles, and neutrons. The ability of an electromagnetic wave (photons) to ionize an atom or molecule depends on its frequency. Radiation on the short-wavelength end of the electromagnetic spectrum - high frequency ultraviolet, x-rays, and gamma rays - is ionizing. Lower-energy radiation such as visible light, microwaves, and radio waves are not. Ionizing radiation is ubiquitous in the environment, and also comes from radioactive materials, x-ray tubes, and particle accelerators. It is invisible and not directly detectable by human senses, and therefore instruments such as Geiger counters are usually required to detect its presence. In some cases it may lead to secondary emission of visible light upon interaction with matter, as in Cherenkov radiation and radioluminescence. Exposure to radiation causes damage to living tissue, and high doses can result in mutation, radiation sickness, cancer, and death.

Alpha, beta, gamma and X-ray radiations are ionizing radiations because they carry enough energy to cause ionizations during their interactions with matter (matter being any material, *i.e*., comprising the body of an animal including human being, the air, and water).

In various embodiments, ionizing radiation is alpha radiation based on alpha particles. Preferably, an alpha emitter according to the present invention is radium. Plutonium is also a preferred alpha emitter according to the present invention.

In various embodiments, ionizing radiation is beta radiation based on beta particles. In preferred embodiments, beta particles according to the present invention are low energy beta particles such as those emitted by tritium (H-3), carbon-14 (C-14), sulphur-35 (S-35), and phosphorus-33 (P-33). In other preferred embodiments, beta particles according to the present invention are high energy beta particles from phosphorus-32 (P-32).

In various embodiments, ionizing radiation is X-ray radiation. In various other embodiments, ionizing radiation is gamma radiation. Furthermore, in various embodiments, ionizing radiation is neutron radiation. Still further, in various embodiments, ionizing radiation is radiation based on high-energy UV rays, some of which are forms of ionizing radiation.

The proximity ligation assay (PLA) is a recently developed tool to visualize protein modifications and (known or suspected) protein-protein interaction on the single protein level [8]. This method is based on the dual proximal binding of two oligonucleotides. In principle primary antibodies from different origin (e.g. mouse and rabbit) bind to their target proteins and are recognized by secondary antibodies which are conjugated to oligonucleotides. After the addition of two further oligonucleotides and ligase, for example, a DNA circle is formed, if the PLA probes are in close proximity. Polymerase and nucleotides are added and the oligonucleotide arm of one of the PLA probes acts as primer for an amplification, preferably a rolling circle amplification using the ligated circular oligonucleotides as template. This reaction generates an amplification product which is detected by fluorescently labeled complementary probes. The two main advantages of establishing a modified PLA in the present invention are the demanding specificity due to the requirement of the double recognition and the high signal strength for straightforward monitoring of single protein events.

An alternative to primary antibodies are aptamers or anticalins, both commonly known in the art. Accordingly, it is envisaged that in the methods of the present invention aptamers or anticalins are applied as an alternative to primary antibodies. As further alternatives to the primary antibodies any protein which is capable of serving as a scaffold for the uptake of binding domains such as CDRs is envisaged to be applied. Accordingly, any of the scaffold molecules described in [9] is envisaged to be applied in the present invention.

The present invention provides a method for determining the level of DNA damage in cells of a subject subjected to ionizing radiation comprising (a) contacting a sample comprising cells obtained from said subject with at least two antibodies which bind to epitopes on the same or different protein(s) which is/are present in DNA repair foci; (b) contacting said sample with at least a first and second proximity probes each comprising a binding moiety with affinity for said antibodies and a nucleic acid acting as a reactive functionality, coupled thereto; (c) allowing the binding moiety of said proximity probes to bind to said antibodies and allowing the nucleic acids of said proximity probes to interact with each other if the proximity probes are in close proximity to each other; and (d) detecting the degree of interaction between the nucleic acids, thereby determining the level of DNA damage.

In the methods of the invention, proteins present in DNA repair foci represent the target analytes. In the present invention, proteins that are "present" in DNA repair foci are those proteins that appear in the cell, preferably cells of a subject after double strand break (DSB) of the DNA. DSBs are generated naturally upon the collapse of replication fork, genome rearrangement by yeast mating type switching, V(D)J recombination, meiosis and exogenous damage. Two main pathways implicated in the repair of a DSB are homologous recombination (HR) and non-homologous end joining (NHEJ).

A cell is preferably from a subject. The subject is preferably a mammal, particularly preferred a human. The term "subject" also includes a horse, a camel, a dog, a cat, a pig, a cow, a goat, a fowl, a rabbit, a mouse, a rat or a zebra fish. However, the cell may also be a cell from a plant, unicellular fungus or yeast.

A cell responds to a DSB by recruiting DNA damage response (DDR) proteins to the chromatin sites near the DSB. Therefore, proteins present in DNA repair foci according to the present invention are DDR proteins recruited to the chromatin sites near a DSB. In other words, proteins present in DNA repair foci according to the present invention are DDR proteins present at the chromatin sites near a DSB.

DDR proteins are described in the art. DDR proteins according to the present invention include the enzymes encoded by the genes described in Table 1 of international patent application WO 2009/001111 A1. Accordingly, Table 1 of WO 2009/001111 represents disclosure content of the present application by way of specific reference made herewith. While the enzymes described in Table 1 of WO 2009/001111 are DNA *repair* enzymes, DDR proteins according to the present invention are DNA damage *response* proteins, which do not only include DNA *repair* proteins (including DNA repair enzymes), but also DNA damage *recognition* proteins (including DNA recognition enzymes). In the present invention, DNA damage *recognition* proteins are proteins recognizing a DNA double strand break.

In various embodiments, proteins present in DNA repair foci according to the present invention are DDR proteins. While the meaning of the term "DNA damage response (DDR) proteins" is well known to the person skilled in the art, preferably proteins present in DNA repair foci according to the present invention are DNA repair proteins. While the meaning of the term "DNA repair proteins" is well known to those of ordinary skill in the art, more preferably DNA repair proteins according to the present invention are those specifically described herein. Also preferred proteins present in DNA repair foci according to the present invention are DNA damage recognition proteins.

Preferred proteins involved in DNA DSB recognition and repair are the proteins shown in the following Tables A and B.

**Table A: Proteins involved in DNA strand break recognition and repair**

| **Protein** | **Molecular Function** | **Pathway** |
|---|---|---|
| **MRE11** | endo/exonuclease | DNA endprocessing |
| **NBS1** | DNA damage signalling | DNA endprocessing |
| **RAD50** | binds to DNA ends | DNA endprocessing |
| **MDC1** | mediator of DNA-damage checkpoint 1 | checkpoint activation |
| **53BP1** | interacts with γ-H2AX | DNA end binding |
| **H2AX** | histone family member X | histone |
| **gamma-H2AX** | phosphorylated H2AX | DNA end binding, histone |
| **ATM** | checkpoint activation, damage signalling | signalling |
| **RNF168** | amplify the RNF8-dependent histone ubiquitination | ubiquitination, DSB repair |
| **BRCA1** | DNA damage repair, | ubiquitination, recombination |
| **RNF8** | ubiquitin ligase | ubiquitination, DSB repair |
| **Rap80** | ubiquitin-binding protein | ubiquitination, DSB repair |

**Table B: Proteins involved in DNA repair**

| **Protein** | **Molecular Function** | **Pathway** |
|---|---|---|
| **Artemis** | nuclease activity together with DNA-PK | non-homologous endjoining |
| **Ku70** | binds to DNA ends | non-homologous endjoining |
| **Ku80** | binds to DNA ends | non-homologous endjoining |
| **DNA-PK** | DNA activated protein kinase | non-homologous endjoining |
| **Ligase-4** | DNA ligase | non-homologous endjoining |
| **XRCC4** | binds to DNA and to DNA ligase 4 | non-homologous endjoining |
| **WRN** | Werner Syndrome, RecQ helicase | recombination |
| **BLM** | Bloom Syndrome, RecQ helicase | recombination |
| **RPA** | replication protein A1 | replication, recombination |
| **Rad51** | homologous recombination | recombination |
| **Rad54** | homologous recombination | recombination |

Accordingly, in various embodiments, preferred proteins present in DNA repair foci according to the present invention are the proteins shown in the above Table A and B.

In various embodiments, the methods of the present invention include the detection of different proteins associating with each other at sites of DNA repair.

Therefore, in various embodiments the methods of the invention comprise binding of at least two antibodies to two different proteins selected from the group of proteins shown in the above Table A and/or B, wherein such two different proteins are proteins associating with each other at sites of DNA repair foci.

Preferred combinations also include combinations of any protein of Table A with any protein of Table B.

Specific examples of preferred combinations are gamma-H2AX and H2AX, gamma-H2AX and NBS1, gamma-H2AX and MRE11, gamma-H2AX and MDC1, gamma-H2AX and RAD50, NBS1 and MRE11, NBS1 and RAD50, NBS1 and MDC1, RAD50 and MRE11, RAD50 and MDC1, MDC1 and MRE11, ATM and phosphor-ATM, or CHK2 and phosphor CHK2NBS1 and MRE11, MDC1 and NBS1, MRE11.

Another preferred combination of different proteins associating with each other at sites of DNA repair foci in accordance with the present invention is the combination of 53BP1 and gamma-H2AX. Phosphorylation of histone H2AX on Ser 139 (gamma-H2AX) is well documented to occur in response to agents that produce double-stranded breaks (Rogakou et al, J. Biol. Chem. 273(10):5858-68, 1998). Previous studies have demonstrated that phosphorylated H2AX is a specific and quantitative marker of double-stranded breaks in nuclear chromatin. Within minutes after ionizing radiation, gamma-H2AX appears in chromatin as discrete foci that co-localize with other cell cycle checkpoint and repair proteins. Gamma-H2AX, the phosphorylated form of H2AX, accumulates at sites of double-stranded DNA breaks and recruits other components of the DNA repair machinery. Break sites in cells can be detected using microscopy and a fluorescence-labeled antibody to gamma-H2AX. DSBs also attract the damage sensor p53-binding protein 1 (53BP1) to the DSB-containing chromatin, because 53BP1 associates with the DSB-surrounding chromatin. Radiation-induced gamma-H2AX and 53BP1 nuclear foci have been shown to be useful markers for detecting radiation exposure after radionuclide incorporation, even for absorbed doses to the blood below 20 mGy.

An also preferred combination of different proteins associating with each other at sites of DNA repair foci in accordance with the present invention is the combination of MRE11 and gamma-H2AX. An also preferred combination of different proteins associating with each other at sites of DNA repair foci in accordance with the present invention is the combination of NBS1 and gamma-H2AX. An also preferred combination of different proteins associating with each other at sites of DNA repair foci in accordance with the present invention is the combination of RAD50 and gamma-H2AX. An also preferred combination of different proteins associating with each other at sites of DNA repair foci in accordance with the present invention is the combination of MDC1 and gamma-H2AX. An also preferred combination of different proteins associating with each other at sites of DNA repair foci in accordance with the present invention is the combination of ATM and gamma-H2AX.

The MRE11 complex is composed of MRE11, RAD50 and NBS1 and is part of the ATM-dependent DNA damage-signaling pathway. In response to DNA damage, the function of the MRE11 complex is regulated by its phosphorylation by the ATM (ataxia-telangiectasia (AT) mutated) protein. Therefore, a preferred combination of different proteins associating with each other at sites of DNA repair foci in accordance with the present invention is the combination of MRE11 and RAD50. An also preferred combination of different proteins associating with each other at sites of DNA repair foci in accordance with the present invention is the combination of MRE11 and NBS1. An also preferred combination of different proteins associating with each other at sites of DNA repair foci in accordance with the present invention is the combination of RAD50 and NBS1.

The MDC1 protein was shown to interact with the MRE11 complex (see international patent application WO 2004/013634). This polypeptide, which was previously termed "MRIP1" (MRE11 complex interacting protein 1), is now called MDC1 (mediator of DNA damage checkpoint protein 1) according to WO 2004/013634. The MDC1 protein was shown to bind to sites of DNA damage and to recruit the MRE11 complex to such sites. Therefore, a preferred combination of different proteins associating with each other at sites of DNA repair foci in accordance with the present invention is the combination of MDC1 and MRE11. An also preferred combination of different proteins associating with each other at sites of DNA repair foci in accordance with the present invention is the combination of MDC1 and RAD50. An also preferred combination of different proteins associating with each other at sites of DNA repair foci in accordance with the present invention is the combination of MDC1 and NBS1.

Furthermore, a preferred combination of different proteins associating with each other at sites of DNA repair foci in accordance with the present invention is the combination of 53BP1 and MRE11. An also preferred combination of different proteins associating with each other at sites of DNA repair foci in accordance with the present invention is the combination of 53BP1 and NBS1. An also preferred combination of different proteins associating with each other at sites of DNA repair foci in accordance with the present invention is the combination of 53BP1 and RAD50. An also preferred combination of different proteins associating with each other at sites of DNA repair foci in accordance with the present invention is the combination of 53BP1 and MDC1.

An also preferred combination of different proteins associating with each other at sites of DNA repair foci in accordance with the present invention is the combination of 53BP1 and ATM. Still further, a preferred combination of different proteins associating with each other at sites of DNA repair foci in accordance with the present invention is the combination of MRE11 and ATM.

The proteins of the above Table A are known to the person skilled in the art, which is demonstrated not at least by the corresponding description of the proteins with respect to, inter alia, their amino acid sequence and deposit given WO 2004/013634.

In various embodiments the methods of the invention comprise binding of at least two antibodies (or aptamers or anticalins) to two different proteins selected from the group of proteins shown in the above Table B.

In various embodiments, the methods of the present invention include the detection of different proteins associating with each other at sites of DNA repair. Therefore, in various embodiments the methods of the invention comprise binding of at least two antibodies to two different proteins selected from the group of proteins shown in the above Table B, wherein such two different proteins are proteins associating with each other at sites of DNA repair foci. A preferred combination of different proteins associating with each other at sites of DNA repair foci in accordance with the present invention is the combination of Ku70 and Ku80. An also preferred combination of different proteins associating with each other at sites of DNA repair foci in accordance with the present invention is the combination of Artemis, DNA-PK, Ligase-4, or XRCC4 and Ku70. An also preferred combination of different proteins associating with each other at sites of DNA repair foci in accordance with the present invention is the combination of Artemis, DNA-PK, Ligase-4, or XRCC4 and Ku80.

In various embodiments the methods of the invention comprise binding of at least two antibodies to two different proteins selected from the group of proteins shown in the above Table C. In various embodiments, the methods of the present invention include the detection of different proteins associating with each other at sites of DNA repair. Therefore, in various embodiments the methods of the invention comprise binding of at least two antibodies to two different proteins selected from the group of proteins shown in the above Table C, wherein such two different proteins are proteins associating with each other at sites of DNA repair foci. A preferred combination of different proteins associating with each other at sites of DNA repair foci in accordance with the present invention is the combination of RAD51 and RAD54. An also preferred combination of different proteins associating with each other at sites of DNA repair foci in accordance with the present invention is the combination of WRN, BLM, or RPA and RAD51. An also preferred combination of different proteins associating with each other at sites of DNA repair foci in accordance with the present invention is the combination of WRN, BLM, or RPA and RAD54.

When describing RAD51 herein, such describing also includes the paralogs of RAD51, namley RAD51 B, RAD51C, RAD51C, XRCC2, and XRCC3. Thus, the above-described combinations for RAD51 include combinations where RAD51 is replaced by one of its paralogs RAD51 B, RAD51C, RAD51C, XRCC2, and XRCC3. The same considerations apply with respect to known paralogs of RAD54, in particular RAD54B paralogs.

In various embodiments the methods of the invention comprise binding of at least two antibodies to two different proteins selected from the group of proteins shown in the above Table D. In various embodiments, the methods of the present invention include the detection of different proteins associating with each other at sites of DNA repair. Therefore, in various embodiments the methods of the invention comprise binding of at least two antibodies to two different proteins selected from the group of proteins shown in the above Table D, wherein such two different proteins are proteins associating with each other at sites of DNA repair foci. A preferred combination of different proteins associating with each other at sites of DNA repair foci in accordance with the present invention is the combination of RNF168 and BRCA1. An also preferred combination of different proteins associating with each other at sites of DNA repair foci in accordance with the present invention is the combination of RNF8 and BRCA1. An also preferred combination of different proteins associating with each other at sites of DNA repair foci in accordance with the present invention is the combination of Rap80 and BRCA1.

The proteins described in the above Tables A to D may be considered as proteins of separate groups, i.e. each of Tables A to D may be considered to describe one separate group of proteins.

The above described combination of proteins within one separate group (i.e., proteins described in one of Tables A to D) does not exclude the combination of proteins from different groups (Tables) described herein. Thus, for example, a preferred combination of different proteins associating with each other at sites of DNA repair foci in accordance with the present invention is the combination of RAD54 (Table C) and ATM (Table A) or the combination of RAD54B paralogs and ATM. Also a preferred combination of different proteins associating with each other at sites of DNA repair foci in accordance with the present invention is the combination of MRE11 (Table A) and DNA-PK (Table B).

As described herein, the method of the present invention comprises a step of contacting a sample comprising cells obtained from a subject that was subjected to ionizing radiation with at least two antibodies which bind to epitopes on the same or different protein(s) which is/are present in DNA repair foci.

An "epitope" is antigenic and thus the term epitope is an "antigenic structure" or "antigenic determinant". The term "epitope" also refers to a site on an antigen (in the context of the present invention, the antigen is any one of the proteins present in a DNA repair foci as described herein) to which the antibody (or aptamer or anticalin) binds. The epitope may be linear or conformational.

As described herein, a "protein which is present in DNA repair foci" may be considered as a "target protein" in accordance with the present invention. Thus, as described herein, the terms "protein which is present in DNA repair foci" and "target protein" are used interchangeably. The same applies to the plural form, i.e. the terms "proteins which are present in DNA repair foci" and "target proteins" are used interchangeably, as described herein.

The method of the present invention comprises a step of contacting a sample comprising cells obtained from a subject that was subjected to ionizing radiation with at least two antibodies which bind to epitopes on the same protein which is present in DNA repair foci. Here, epitopes on the same protein means *different* epitopes on the same protein given that at least two antibodies are to be used in corresponding embodiments, *i.e*. embodiments where at least two antibodies bind to epitopes on the target protein. That is, the at least to antibodies bind to at least two different epitopes on the target protein. In other words, each antibody used in corresponding embodiments binds to a separate epitope on the target protein.

In various embodiments, the method of the present invention comprises a step of contacting a sample comprising cells obtained from a subject that was subjected to ionizing radiation with at least two antibodies (or aptamers or anticalins) which bind to epitopes on different proteins which are present in DNA repair foci. Here, the epitopes are different per se because of the different nature of the proteins. Preferably, two different proteins are the target proteins in embodiments where at least two antibodies bind to epitopes on different target proteins. Accordingly, two antibodies bind to two different target proteins. That is, in various embodiments, the method of the present invention comprises a step of contacting a sample comprising cells obtained from a subject that was subjected to ionizing radiation with two antibodies which bind to epitopes on two different proteins which are present in DNA repair foci.

As described herein, the methods of the present invention include the detection of proteins associating with gamma-H2AX at sites of DNA repair. Therefore, in various embodiments the method of the present invention comprises a step of contacting a sample comprising cells obtained from a subject that was subjected to ionizing radiation with two antibodies wherein one antibody binds to epitopes on gamma-H2AX and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on 53BP1 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on MDC1 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on MRE11 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on NBS1 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on RAD50 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on Ku70 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on Ku80 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on RAD51 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on RAD54 and the other binds to epitopes on another, different, protein present in DNA repair foci according to the present invention.

In various embodiments the method of the present invention comprises a step of contacting a sample comprising cells obtained from a subject that was subjected to ionizing radiation with two antibodies wherein one antibody binds to epitopes on BRCA1 and the other binds to epitopes on another, different, protein present in DNA repair foci according to the present invention.

The method of the present invention comprises a step of (a) contacting a sample comprising cells obtained from a subject that was subjected to ionizing radiation with at least two antibodies which bind to epitopes on the same or different protein(s) which is/are present in DNA repair foci, and a step of (b) contacting said sample with at least a first and second proximity probes each comprising a binding moiety with affinity for said antibodies and a nucleic acid acting as a reactive functionality, coupled thereto. In various embodiments, the said steps (a) and (b) may be carried out simultaneously. In various other embodiments, the said steps (a) and (b) may be changed, i.e. step (b) is carried out prior to step (a).

The method of the present invention comprises a step of allowing the binding moiety of the proximity probes according to the present invention to bind to the antibodies (or aptamers or anticalins) binding to epitopes on the same or different target proteins according to the present invention, thereby allowing the nucleic acids of the proximity probes to interact with each other if the proximity probes are in close proximity to each other. The proximity probes are in close proximity to each other if the antibodies (or aptamers or anticalin) bind to epitopes on the same target protein. Thus, in various embodiments the method of the present invention comprises a step of allowing the binding moiety of the proximity probes according to the present invention to bind to the antibodies (or aptamers or anticalins) binding to epitopes on the same target protein according to the present invention, thereby allowing the nucleic acids of the proximity probes to interact with each other.

Furthermore, the proximity probes are in close proximity to each other if the antibodies or aptamers or anticalins) bind to epitopes on different target proteins. This is because the target proteins according to the methods of the present invention are proteins present at the sites of DNA repair, preferably at sites of DNA repair of DSBs. Therefore, different proteins present in DNA repair foci according to the present invention (or different target proteins according to the present invention) are *per* se in close proximity to each other. Thus, in various embodiments the method of the present invention comprises a step of allowing the binding moiety of the proximity probes according to the present invention to bind to the antibodies (or aptamers or anticalins) binding to epitopes on different target proteins according to the present invention, thereby allowing the nucleic acids of the proximity probes to interact with each other.

As described herein, the target proteins according to the present invention are preferably target proteins associating with each other at sites of DNA damage or DNA repair. Thus, in various embodiments the method of the present invention comprises a step of allowing the binding moiety of the proximity probes according to the present invention to bind to the antibodies binding to epitopes on different target proteins associating with each other at sites of DNA damage or DNA repair, thereby allowing the nucleic acids of the proximity probes to interact with each other.

In various embodiments, proteins associating with each other at sites of DNA damage or DNA repair means that different proteins present at sites of DNA repair (or different proteins present in DNA repair foci) are functionally associated with each other. That is, for example, in response to DNA damage, the function of the MRE11 complex is regulated by its phosphorylation by the ATM protein and by related kinases such as ATR and DNA-PK. Therefore, in various embodiments, proteins associating with each other at sites of DNA damage or DNA repair in accordance with the present invention are two different proteins present at sites of DNA damage or DNA repair, wherein one protein is regulated by the activity of the other protein. Preferably, the said one protein is regulated by its phosphorylation by the kinase activity of the other protein.

In various embodiments, proteins associating with each other at sites of DNA damage or DNA repair means that different proteins, preferably two different proteins, are jointly involved in DNA repair signaling. In various embodiments, proteins associating with each other at sites of DNA damage or DNA repair means that different proteins, preferably two different proteins, are jointly involved in DSB repair pathways, *i.e*. in non-homologous end joining (NHEJ) or homologous recombination (HR) repair.

Spatial and temporal recruitment of mediator and DNA repair proteins to the irradiation-induced foci is necessary for efficient DNA repair to occur. In various embodiments, proteins associating with each other at sites of DNA damage or DNA repair means two different proteins, wherein the presence or activity of one of both proteins at the sites of DNA damage or DNA damage causes or is involved in recruitment of the other protein to the sites of DNA damage or DNA damage or DNA repair. In other words, one protein of the two different proteins is required for the active recruitment of the other protein to the sites of DNA damage or DNA repair.

In various embodiments, proteins associating with each other at sites of DNA damage or DNA repair means two different proteins, wherein one protein has the activity of binding to the other protein at the sites of DNA damage or DNA repair.

In various embodiments, proteins associating with each other at sites of DNA damage or DNA repair means different proteins, preferably two different proteins, wherein said different proteins share the characteristic that they are chromatin-associated proteins, preferably chromatin-associated proteins that are involved in DNA repair.

Also any other kind of association of proteins present in DNA repair foci according to the present invention is contemplated by the present invention, including physical association of target proteins according to the present invention.

In the present invention, the terms "present in DNA repair foci" and "present at sites of DNA repair" may be used interchangeably. Both terms include the presence of DNA damage response proteins according to the present invention, *i.e*. "DNA repair foci" according to the present invention include the presence of DNA damage response proteins according to the present invention, and "sites of DNA repair" according to the present invention also include the presence of DNA damage response proteins according to the present invention.

In the present invention, DNA damage is preferably DNA damage comprising one or more double strand breaks (DSBs), or at least one DSB, in the DNA of a cell, preferably a cell of a subject. Furthermore, in the present invention such a DSB is preferably caused by ionizing radiation. Therefore, in various embodiments of the present invention DNA damage means one or more DSBs, or at least one DSB, in the DNA of a subject after subjecting the subject to ionizing radiation. Ionizing radiation is known in the art as a cause of DNA damage, in particular DSBs. Therefore, there is no doubt that DNA damage (in particular DSBs) determined according to the present invention in a subject that was subjected to ionizing radiation is DNA damage (in particular DSBs) caused by said ionizing radiation.

In the present invention, DNA repair means repair of one or more double strand breaks (DSBs), or at least one DSB, in the DNA of a cell of a subject. Furthermore, in the present invention such a DSB is preferably caused by ionizing radiation. Therefore, in various embodiments of the present invention DNA repair means repair of one or more DSBs, or at least one DSB, in the DNA of a subject after subjecting the subject to ionizing radiation.

The method of the present invention comprises a step of detecting the degree of interaction between the nucleic acids of the binding moieties of the first and second proximity probes according to the present invention, thereby determining the level of DNA damage in cells of a subject subjected to ionizing radiation. In various embodiments, the degree of interaction is reflected in the detectable signal generated by the interaction of the nucleic acids. This signal can be detected by standard means available to the skilled person from the art.

In various embodiments, the cells are permeabilized prior to contacting the sample comprising the cells with the antibodies that specifically bind to epitopes on the same or different protein(s) present in DNA repair foci according to the present invention.

As described herein, the nucleic acids of the first and second proximity probes act as reactive functionalities, describing their capability of pair wise reacting with each other. As described herein, the reactive functionality of a proximity probe is comprised of a nucleic acid coupled to the binding moiety. This coupling may be a covalent cross-linking or non-covalent, as exemplified by a streptavidin-biotin coupling. As proximity probes are brought in close proximity to each other according to the methods of the present invention, the coupled nucleic acids (reactive functionalities) are also brought in vicinity of each other. The proximity of these nucleic acids is used to promote various detectable interactions between these nucleic acids, which interaction is detected or determined in the methods of the present invention. In various embodiments, detecting the degree of interaction between the nucleic acids is detecting the amount of nucleic acid interaction. In various embodiments, the interacted nucleic acids are amplified before detecting the degree of interaction between the nucleic acids of the binding moieties of the first and second proximity probes according to the present invention.

In the present invention, one of the binding moieties of one of the two proximity probes will typically have a certain nucleic acid sequence coupled to it by its 5'-end while the other binding moiety of the other proximity probe has a nucleic acid sequence coupled to it by its 3'-end. The 5'-proximity probe thus has a free 3'-hydroxyl capable of reacting with the 5'-phosphate of the second binding moiety through interaction. As described herein, each nucleic acid coupled to a proximity probe according to the present invention is a unique nucleic acid attached thereto.

In the methods of the present invention, the proximity probes are added at a low concentration. The concentration of the proximity probe is set so low that the proximity probes will not react with each other to any great extent when the proximity probes are not near each other. But when two or more the proximity probes bind to the antibodies binding to the target protein(s) the high local concentration promotes interaction between the probes, resulting in a detection signal.

The product of the ligation reaction will in most cases exist at a low concentration so that an amplification step may be needed in order to detect it. The resulting ligation between these nucleic acids is readily amplifiable by several reactions, such as polymerase chain reaction (PCR), strand displacement amplification, NASBA, RNA transcription, invader assay. These amplification reactions are thus designed only to give a product when the two nucleic acids have been cross-linked by ligation.

In various embodiments, a lot of ligation product is produced. It can then be directly analyzed without the need of an amplification step. Several methods can then be employed. For example, the first of the two united nucleic acid sequences can be designed to contain a sequence capable of binding through hybridization to an immobilized oligonucleotide probe. Upon binding the oligonucleotide probe, the second nucleic acid sequence will also bind since the two are now linked by the ligation. All non bound sequences can be washed off and a labeled hybridization probe specific for the second sequence will bind and detect the ligation product.

The two united nucleic acids may be analyzed in a hybridization-based assay where the united sequence elements bind cooperatively and thereby much stronger than non-united ones to an oligonucleotide probe. Such probes may be constructed in DNA microarrays to selectively bind nucleic acids with united sequence elements.

In various embodiments, the interaction of the nucleic acids coupled to the binding moieties is through hybridization to a common splint template and ligation of the nucleic acid ends. The splint template is an oligonucleotide and is also called DNA splint or template strand. In corresponding embodiments, the ligation reaction of the nucleic acid ends is dependent on the hybridized splint template when using a DNA ligase enzyme such as T4 DNA ligase. The two nucleic acids to be ligated must hybridize to the template with the 3'-end of one oligonucleotide lined up to the 5'-phosphate of the other.

When the ligation template is added to the reaction it may hybridize to a pair of nearby proximity probes and promote ligation. But if each proximity probe nucleic acid has bound a ligation template no ligation will occur since no single ligation template spans pairs of ligatable ends. If the ligation template is added at a high concentration the probability for a one to one hybridization will be greater. But if the proximity probes have bound their target, the high local concentration of their nucleic acids will favor a generation of appropriate ligation substrates. This mechanism serves to promote ligation in the presence of target protein(s), and to inactivate proximity probes in the absence of antigens, improving the signal to noise ratio.

The splint template should be designed so as to not give rise to any false amplification products. If incorrectly designed the ligation template may be used by the amplification enzyme, such as DNA polymerase, as a template for polymerization. This extension, templated by the ligation template, may give rise to false products. To overcome this problem, DNA oligonucleotides may be ligated with an RNA oligonucleotide as template using the T4 DNA ligase enzyme. Taq DNA polymerase cannot use RNA as a template for polymerization, hence no false PCR products can arise from polymerization on such a ligation template. A DNA splint with two short hybridization regions (such as 10+10) also works like the RNA splint. But since the hybridization is weak they will not template polymerization at the high temperature of the PCR, due to the low melting temperature. Alternative means to react the conjugated nucleic acids is through the T4 RNA ligase which does not need any template strand, or to use chemical ligation to join ends that cannot prime polymerization reactions.

The DNA splint or splint template described above is a "free splint oligonucleotide", which is different from a "binding splint", which is described herein elsewhere. As described herein, in embodiments using a "free splint oligonucleotide" the splint is added at the ligation step, subsequent to the binding step, and therefore binding and dissociation of individual probes occurs independently.

As described herein, in various embodiments the method of the invention comprises binding of at least two antibodies to epitopes on different target proteins, preferably binding of two antibodies to epitopes on two different target proteins. In these embodiments, the degree of interaction between the nucleic acids of the binding moieties of the first and second proximity probes according to the present invention is indicative of the interaction between the different target proteins, preferably two different target proteins. Therefore, in various embodiments the method of the present invention comprises a step of detecting the interaction between different target proteins, preferably two different target proteins, thereby determining the level of DNA damage according to the present invention.

The method of the present invention depends upon detecting the presence of a target protein in a sample by detecting the interaction between two proximity probes, when such probes are bound to antibodies, which in turn are bound to target protein(s). As described herein, the interaction between the two proximity probes (or more specifically, between their respective nucleic acids) is thus proximity dependent. The binding of the two detection probes, together, on the target protein-bound antibodies brings them into close proximity, such that they (or more particularly, their nucleic acids) may interact. Accordingly, by detecting their interaction (or conjugation), the target protein may be detected.

In various embodiments, the method of the present invention comprises contacting the sample comprising cells obtained from a subject subjected to ionizing radiation with a third proximity probe in addition to the first and second proximity probe according to the present invention. Like the first and second proximity probe, the third proximity probe comprises a binding moiety with affinity for an antibody which binds to an epitope on the same or different protein(s) which is/are present in DNA repair foci, as well as a third nucleic acid acting as a reactive functionality, coupled thereto. As described, the nucleic acid of the third proximity probe contains regions of complementarity for the ends of the nucleic acid domains of the first and second proximity probes and is thereby able to act as a splint to template their ligation. Accordingly, in corresponding embodiments which include the use of a third proximity probe, the nucleic acid of the first proximity probe has a 3' free nucleic acid (A), the nucleic acid of the second proximity probe has a 5' free nucleic acid (B), and the nucleic acid of the third proximity probe has both 3' and 5' free nucleic acids (C), wherein the 3'-end of (A) interacts with the 5' end of (C), and the 3'-end of (C) interacts with the 5'-end of (B). By coupling the ligation-templating splint oligonucleotide to a further proximity probe, the splint is co-localized within the sample with the first and second proximity probes. This template splint is called "binding splint" and its use further improves the sensitivity and specificity of the assay. Furthermore, since the binding splint (of the third proximity probe) is added to the reaction at the same time as the first and second proximity probes, the three proximity probes can interact immediately when brought into close proximity through binding to their respective antibodies. This produces an avidity effect by reducing the dissociation rate of the target protein-antibody-probe complex, thereby acting to stabilize the complex.

In various embodiments, the proximity probes may interact by being conjugated, or joined to one another, as described herein, and the binding splint assists in or mediates this interaction (conjugation). The conjugation may be detected by detecting the conjugation product (interaction product).

The term "detecting" is used broadly herein to include any means of determining the presence of a target protein according to the present invention (*i.e*., if it is present or not) or any form of measurement of the target protein. Thus "detecting" may include determining, measuring, assessing or assaying the presence or absence or amount or location of a target protein according to the present invention in any way. Quantitative and qualitative determinations, measurements or assessments are included, including semi-quantitative. Such determinations, measurements or assessments may be relative, for example when two or more different target proteins in a sample are being detected, or absolute. As such, the term "quantifying" when used in the context of quantifying a target protein(s) in a sample can refer to absolute or to relative quantification. Absolute quantification may be accomplished by inclusion of known concentration(s) of one or more control analytes/proteins and/or referencing the detected level of the target protein with known control analytes/proteins (*e.g*., through generation of a standard curve). Alternatively, relative quantification can be accomplished by comparison of detected levels or amounts between two or more different target analytes to provide a relative quantification of each of the two or more different target proteins, *i.e*., relative to each other.

Proximity probe based assays, such as that of the present invention, have found particular utility in the detection of proteins or polypeptides. While the DNA damage response proteins according to the present invention are the "targets" to be detected by the methods of the present invention, the analyte(s) to be applied to the methods of the present invention may comprise target protein complexes, which may be a homo- or hetero-multimer target protein complex. Aggregates of target proteins may also be target analytes, for example aggregates of the same protein or different proteins.

A sample comprising cells obtained from a subject subjected to ionizing radiation is used in the methods according to the present invention. Here, all biological and clinical samples are included, *e.g*. any cell or tissue sample of an organism, or any body fluid or preparation derived therefrom, as well as samples such as cell cultures, cell preparations, cell lysates etc. Representative samples thus include any cell which may contain a protein according to the present invention, *i.e*. proteins present in DNA repair foci. Representative samples thus include whole blood and blood-derived products such as plasma, serum and buffy coat, blood cells, urine, faeces, cerebrospinal fluid or any other body fluids (e.g. respiratory secretions, saliva, milk, etc.), tissues, biopsies, cell cultures, cell suspensions, conditioned media or other samples of cell culture constituents, etc. Preferably, the sample is a tissue sample. Also preferred is whole blood as a sample to be used in the methods of the present invention.

In various embodiments, the sample is pre-treated in any convenient or desired way to prepare for use in the methods of the invention, for example by cell lysis or purification, isolation of the target protein(s), etc. In various embodiments, the tissue sample is processed so as to enrich a cell culture. Preferably, the enriched cells are epithelial cells. Methods for culturing and enrichment of such cells are well known to the person skilled in the art. Accordingly, in various embodiments the sample is a cell culture generated from a tissue sample obtained from a subject.

The proximity probes for use in the methods of the present invention comprise a binding moiety and a nucleic acid, and are in effect detection probes which bind to an antibody, which in turn binds a target protein according to the present invention, the binding of which may be detected (to detect the target protein) by means of detecting the interaction which occurs between the nucleic acids thereof upon such binding. Accordingly the proximity probes may be viewed as nucleic acid-tagged affinity ligands or binding partners for their respective target protein-binding antibodies, the antibody being the affinity binding partner, and the nucleic acid domain the nucleic acid tag. The nucleic acid is coupled to the binding moiety and this "coupling" or connection may be by any means known in the art, and which may be desired or convenient and may be direct or indirect, *e.g.* via a linking group. For example, the domains may be associated with one another by covalent linkage (e.g. chemical cross-linking) or by non-covalent association, e.g. via streptavidin-biotin based coupling (biotin being provided on one domain and streptavidin on the other).

The binding moiety may be any binding partner for an antibody binding a target protein according to the present invention. The binding moiety indirectly binds to the target protein via the antibody as intermediary molecule or binding partner, which binds to the target protein. The binding moiety binds to said intermediary molecule (binding partner). Particularly, the antibody or the intermediary binding partner is a specific binding partner for the target protein. The antibody as a specific binding partner is capable of binding specifically to the target protein such that the antibody binds to the target protein with greater affinity and/or specificity than to other components in the sample. Thus binding to a target protein may be distinguished from non-target analytes; the specific binding partner (antibody) either does not bind to non-target analytes or does so negligibly or non-detectably or any such non-specific binding, if it occurs, may be distinguished. The binding between the target protein and its binding partner (antibody) is typically non-covalent.

The binding moiety may be selected to have a high binding affinity for its respective antibody. By high binding affinity is meant a binding affinity of at least about 10⁻⁴ M, usually at least about 10⁻⁶ M or higher, e.g., 10⁻⁹ M or higher. The binding moiety may be any of a variety of different types of molecules, so long as it exhibits the requisite binding affinity for its respective antibody when present as part of the proximity probe. In various embodiments, the binding moiety may be a ligand that has medium or even low affinity for its antibody, *e.g*., less than about 10⁻⁴ M.

In various embodiments, binding moieties according to the present invention are antibodies. If the binding moieties are antibodies, the antibodies which bind to epitopes on the same or different protein(s) which is/are present in DNA repair foci according to the present invention may be called primary antibodies. In this case, the antibodies of the proximity probes according to the present invention may be called secondary antibodies. Accordingly, secondary antibodies according to the present invention are those having affinity for the aforementioned primary antibodies. Thus, in various embodiments the proximity probes according to the present invention comprise a secondary antibody with affinity for a respective primary antibody and a nucleic acid acting as a reactive functionality, coupled thereto.

In various embodiments, binding moieties according to the present invention are polynucleic acid aptamers. Polynucleic acid aptamers may be RNA oligonucleotides which may act to selectively bind proteins, much in the same manner as a receptor or antibody (Conrad et al., Methods Enzymol. (1996), 267:336-367).

In various embodiments, the binding moiety may be one that includes a moiety that can be covalently attached to the nucleic acid acting as reactive functionality without substantially abolishing the binding affinity of the binding moiety to its antibody.

Antibodies to be used in the present invention include binding fragments and derivatives or mimetics thereof, as long as such fragments, derivatives and mimetics bind to the target protein. For example, antibody fragments, such as Fv, F(ab)2 and Fab may be prepared by cleavage of the intact protein, e.g. by protease or chemical cleavage. Also of interest are recombinantly or synthetically produced antibody fragments or derivatives, such as single chain antibodies or scFvs, or other antibody derivatives such as chimeric antibodies or CDR-grafted antibodies, as long as such recombinantly or synthetically produced antibody fragments bind to a target protein according to the present invention. Such antibody fragments or derivatives generally include at least the VH and VL domains of the subject antibodies in order to bind to a target protein according to the present invention. Such antibody fragments, derivatives or mimetics of the subject invention may be readily prepared using any convenient methodology, such as the methodology disclosed in US 5,851,829 and 5,965,371.

The above described antibodies, fragments, derivatives and mimetics thereof may be obtained from commercial sources and/or prepared using any convenient technology, where methods of producing polyclonal antibodies, monoclonal antibodies, fragments, derivatives and mimetics thereof, including recombinant derivatives thereof, are known to those of the skill in the art.

In various embodiments, the antibodies are polyclonal antibodies. In various other embodiments, the antibodies are monoclonal antibodies. In various embodiments, one antibody to be used in the methods of the present invention may be a polyclonal antibody, while the other antibody may be a monoclonal antibody. As such, the antibodies to be used in the present invention may be either monoclonal or polyclonal antibodies.

In addition to antibody-based binding, a target protein according to the present invention may also be bound by a lectin, a soluble cell-surface receptor or derivative thereof, an affibody or any combinatorially derived protein or peptide from phage display or ribosome display or any type of combinatorial peptide or protein library. In various embodiments, combinations of any of the above may be used.

The binding sites on the proteins according to the present invention for the respective protein-binding domains of the antibodies in a set may be the same or different. Thus, for example in the case of a homomeric protein complex or aggregate comprising two or more identical subunits or protein constituents, the at least two antibodies according to the present invention may be the same. Where the protein to be bound by an antibody according to the present invention is a single molecule or comprises different sub-units or constituents (*e.g*., a heteromeric complex or an aggregate of different proteins), the antibodies to be used in the methods of the present invention will be different.

The length of the nucleic acid of the binding moieties of the proximity probes according to the present invention can be constructed to span varying molecular distances.

As described herein, the proximity probe binds to the target protein indirectly because the target protein is first bound by an antibody, which functions as a specific binding partner (or intermediary molecule or affinity ligand). The binding moieties of the proximity probes according to the present invention bind to the respective antibodies acting as the specific binding partner. This enables the design of proximity probes as universal reagents. That is a universal proximity probe set may be used to detect different proteins by binding to the Fc regions of the various different antibodies binding to the target proteins.

The nucleic acids of the first and second proximity probes may be regarded as the nucleic acid "tags" which interact to form a detectable product, which may be detected to report the detection of the target protein. The nucleic acids according to the present invention are thus regarded as reactive nucleic acid functionalities, which interact to provide the signal by means of which the target protein is detected (more particularly to form a signal-giving product). In other words, the nucleic acids may be regarded as "detection tags", which interact to form a "detectable" tag or product. When two or more proteins are present in the same sample they may be detected simultaneously using two or more sets of proximity probes, each set of proximity probes being designed to form on interaction a unique nucleic acid sequence "detectable tag". These unique "detectable tags" may be detected and quantified (optionally after amplification) separately using methods well known in the literature including liquid chromatography, electrophoresis, mass spectrometry, DNA array technology and multi-colour real-time PCR.

In the methods of the present invention, the nucleic acids acting as reactive functionalities of the first and second proximity probes may be conjugated together. As explained further below, this conjugation may be direct, *i.e*. the respective nucleic acids may be directly joined to one another, or it may be indirect, *i.e*. the respective nucleic acid domains may be joined indirectly *e.g*. by joining each to one of the two ends of a further intermediary nucleic acid molecule (*e.g*. oligonucleotide). This "conjugation" or "interaction" is mediated by the binding splint, which is provided as the nucleic acid of the third proximity probe, as described herein. The conjugation results in the formation of a new nucleic acid molecule, or sequence, which may be detected.

The nucleic acids acting as reactive functionalities are "tagged" to the binding moieties of the proximity probes. Each binding moiety has affinity for only one antibody, the latter specifically binding to one target protein. If the antibody binding to a target protein is a polyclonal antibody, each antibody of the heterogenous antibody population is tagged with the same nucleic acid. Likewise, if the antibody binding to a target protein is a monoclonal antibody, each antibody of the homogenous antibody population is tagged with the same nucleic acid. As described herein, the nucleic acid of the third proximity probe is a "binding splint" which hybridizes to the nucleic acids of the first and second proximity probes, enabling their conjugation (or interaction).

The nucleic acids according to the present invention may be a single stranded nucleic acid molecule (*e.g*. an oligonucleotide), a partially double stranded and partially single stranded molecule, or a double stranded molecule that includes of a region that is double stranded and a region where the two nucleic acid strands are not complementary and therefore single stranded. As such, in various embodiments, the nucleic acid is made up of a single stranded nucleic acid. In other embodiments, the nucleic acid may be made up of two partially complementary nucleic acid strands, where the two strands include a hybridized region and non-hybridized region.

The nucleic acids acting as reactive functionalities according to the present invention are generally of a length sufficient to allow splint-mediated interaction between nucleic acids of different proximity probes when bound to the respective antibodies. This applies to both "free splint oligonucleotides" and "binding splint oligonucleotides" described herein. While in the present invention nucleic acids are usually in the range of between about 8 up to about 1000 nucleotides in length, in various embodiments they may range from about 8 to about 500 nucleotides in length. Preferably, the nucleic acids range from about 8 to about 250 nucleotides in length, more preferably from about 8 to about 160 nucleotides in length, and still more preferably from about 12 to about 150 nucleotides in length. Even more preferably, the nucleic acids range from about 14 to about 130 nucleotides in length. In further preferred embodiments, the nucleic acids range from about 16 to about 110 nucleotides in length, from about 8 to about 90 nucleotides in length, from about 12 to about 80 nucleotides in length, from about 14 to about 75 nucleotides in length, from about 16 to about 70 nucleotides in length, or from about 16 to about 60 nucleotides in length. In further preferred embodiments, the nucleic acids range in length from about 10 to about 80 nucleotides in length, from about 12 to about 75 nucleotides in length, from about 14 to about 70 nucleotides in length, from about 34 to about 60 nucleotides in length, or any length between the stated ranges. In even more preferred embodiments, the nucleic acids according to the present invention are not more than about 25, 26, 27, 28, 29, 30, 31, 32, 33, 34, 35, 36, 37, 38, 39, 40, 41, 42, 44, 46, 50, 55, 60, 65, or 70 nucleotides in length.

The nucleic acids may be made up of ribonucleotides and/or deoxyribonucleotides as well as synthetic nucleotide residues that are capable of participating in Watson-Crick type or analogous base pair interactions. Thus, the nucleic acid may be DNA or RNA or any modification thereof, *e.g*. PNA or other derivatives containing non-nucleotide backbones. The sequence of the nucleic acid of the first and second proximity probes (*i.e*., the "detection" nucleic acids) may be chosen or selected with respect to the binding splint provided on the third proximity probe. Thus, the sequence is not critical as long as the first and second domains may hybridize to the third domain (binding splint). However, the sequences should be chosen to avoid the occurrence of hybridization events other than between the nucleic acids of the first and second proximity probes with that of the third proximity probe. Once the sequence is selected or identified, the nucleic acids may be synthesized using any convenient method known in the art.

The two components of the proximity probe are joined together either directly through a bond or indirectly through a linking group. Where linking groups are employed, such groups may be chosen to provide for covalent attachment of the nucleic acid and binding moieties through the linking group, as well as maintain the desired binding affinity of the binding moiety for its respective antibody. Linking groups of interest may vary widely depending on the binding moiety. The linking group, when present, is in many embodiments biologically inert. A variety of linking groups are known to those of skill in the art and find use in the subject proximity probes. In representative embodiments, the linking group is generally at least about 50 Daltons, usually at least about 100 Daltons and may be as large as 1000 Daltons or larger, for example up to 1,000,000 Daltons if the linking group contains a spacer, but generally will not exceed about 500 Daltons and usually will not exceed about 300 Daltons. Generally, such linkers will comprise a spacer group terminated at either end with a reactive functionality capable of covalently bonding to the nucleic acids or binding moieties. Spacer groups of interest may include aliphatic and unsaturated hydrocarbon chains, spacers containing heteroatoms such as oxygen (ethers such as polyethylene glycol) or nitrogen (polyamines), peptides, carbohydrates, cyclic or acyclic systems that may possibly contain heteroatoms. Spacer groups may also be comprised of ligands that bind to metals such that the presence of a metal ion coordinates two or more ligands to form a complex. Specific spacer elements include: 1,4-diaminohexane, xylylenediamine, terephthalic acid, 3,6-dioxaoctanedioic acid, ethylenediamine-N,N-diacetic acid, 1,1'-ethylenebis(5-oxo-3-pyrrolidinecarboxylic acid), 4,4'-ethylenedipiperidine. Potential reactive functionalities include nucleophilic functional groups (amines, alcohols, thiols, hydrazides), electrophilic functional groups (aldehydes, esters, vinyl ketones, epoxides, isocyanates, maleimides), functional groups capable of cycloaddition reactions, forming disulfide bonds, or binding to metals. Specific examples include primary and secondary amines, hydroxamic acids, N-hydroxysuccinimidyl esters, N-hydroxysuccinimidyl carbonates, oxycarbonylimidazoles, nitrophenylesters, trifluoroethyl esters, glycidyl ethers, vinylsulfones, and maleimides. Specific linker groups that may find use in the subject proximity probes include heterofunctional compounds, such as azidobenzoyl hydrazide, N-[4-(p-azidosalicylamino)butyl]-3'-[2'-pyridyldithio]propionamid), bis-sulfosuccinimidyl suberate, dimethyladipimidate, disuccinimidyltartrate, N-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, and succinimidyl-4-[N-maleimidomethyl]cyclohexane-1-carboxylate, 3-(2-pyridyldithio)propionic acid N-hydroxysuccinimide ester (SPDP), 4-(N-maleimidomethyl)-cyclohexane-1-carboxylic acid N-hydroxysuccinimide ester (SMCC), and the like.

The proximity probes employed in the subject methods may be prepared using any convenient method. In representative embodiments, nucleic acid domains may be conjugated to the binding moieties, either directly or through a linking group. The components can be covalently bonded to one another through functional groups, as is known in the art, where such functional groups may be present on the components or introduced onto the components using one or more steps, *e.g*. oxidation reactions, reduction reactions, cleavage reactions and the like. Functional groups that may be used in covalently bonding the components together to produce the proximity probe include: hydroxy, sulfhydryl, amino, and the like. The particular portion of the different components that are modified to provide for covalent linkage may be chosen so as to not substantially adversely interfere with that component's desired binding affinity for the respective antibody. Where necessary and/or desired, certain moieties on the components may be protected using blocking groups, as is known in the art, see *e.g*. Green & Wuts, Protective Groups in Organic Synthesis (John Wiley & Sons) (1991). Methods for producing nucleic acid/antibody conjugates are well known to those of skill in the art. See *e.g*. U.S. Patent No. 5,733,523, the disclosure of which is herein incorporated by reference.

In other embodiments, proximity probes may be produced using in vitro protocols that yield nucleic acid-protein conjugates, *i.e*. molecules having nucleic acids, *e.g*. coding sequences, covalently bonded to a protein, *i.e*. where the binding moiety is produced in vitro from vectors which encode the proximity probe. Examples of such in vitro protocols of interest include: RepA based protocols (see *e.g*., Fitzgerald, Drug Discov. Today (2000) 5:253-258 and WO 98/37186), ribosome display based protocols (see *e.g*., Hanes et al., Proc. Natl. Acad. Sci. USA (1997) 94:4937-42; Roberts, Curr. Opin. Chem. Biol. (1999) Jun; 3:268-73; Schaffitzel et al., J. Immunol. Methods (1999) Dec 10; 231:119-35; and WO 98/54312), etc.

The nucleic acid of the third proximity probe is a binding splint oligonucleotide which functions to mediate the interaction between the nucleic acids of the first and second proximity probes (*i.e*. the "detection" domains). As noted above, the use of a splint in proximity ligation assays is known in the art. The splints according to the present invention may accordingly be viewed as a "connector" oligonucleotides which act to connect or "hold together" the first and second proximity probes, such that they may interact by means of a ligation reaction and be conjugated together.

In particular, a splint according to the present invention hybridizes with the nucleic acids of the first and second proximity probes. More particularly, the splint hybridizes (anneals) simultaneously with the nucleic acids of at least the first and second proximity probes. This hybridization of the nucleic acids of all of the set of proximity probes to each other increases the avidity of the probe-antibody-target protein complex upon binding to the target protein. This avidity effect contributes to the sensitivity of the assay by supporting the formation of signal-giving proximity probe-antibody-target protein complexes.

The term "hybridization" or "hybridizes" as used herein refers to the formation of a duplex between nucleotide sequences which are sufficiently complementary to form duplexes via Watson-Crick base pairing. Two nucleotide sequences are "complementary" to one another when those molecules share base pair organization homology. "Complementary" nucleotide sequences will combine with specificity to form a stable duplex under appropriate hybridization conditions. For instance, two sequences are complementary when a section of a first sequence can bind to a section of a second sequence in an anti-parallel sense wherein the 3'-end of each sequence binds to the 5'-end of the other sequence and each A, T(U), G and C of one sequence is then aligned with a T(U), A, C and G, respectively, of the other sequence. RNA sequences can also include complementary G=U or U=G base pairs. Thus, two sequences need not have perfect homology to be "complementary" in the present invention. Usually two sequences are sufficiently complementary when at least about 85%, preferably at least about 90%, and more preferably at least about 95% of the nucleotides share base pair organization over a defined length of the molecule. The nucleic acids of the first and second proximity probes thus contain a region of complementarity for the nucleic acid of the third proximity probe, and conversely the nucleic acid of the third proximity probe contains regions of complementarity for each of the nucleic acids of the first and second proximity probes.

The regions of complementarity (*i.e*. hybridization regions) may have a length in the range of 4-30 bp, more preferably in the range of 6-20 bp, still more preferably in the range of 6-18 bp, even more preferably in the range of 7-15 bp, and most preferably in the range of about 8-12 bp.

The splint nucleic acid in accordance with the present invention (*i.e*., "free splint nucleic acid" and "binding splint nucleic acid") is generally of a length sufficient to provide for the above described simultaneous binding of nucleic acids of the first and second probes. In various embodiments, the splint oligonucleotides used in the present invention range in length from about 6 to about 500 nucleotides, preferably from about 20 to about 40 nucleotides, and more preferably from about 25 to about 30 nucleotides.

As noted above, the interaction or conjugation between the nucleic acid domains of the first and second proximity probes is a joining of the respective nucleic acids by ligation. This joining may preferably be a ligation, particularly a template-directed ligation. In such a case, it will clearly be understood that the ligation template will be provided by a splint as described herein. Such a ligation may be carried out using a ligase enzyme.

Thus, in a preferred embodiment of the method of the invention, the nucleic acids of the first and second probes are ligatable by means of a reaction templated by a hybridized splint (free splint or binding splint), said nucleic acids are ligated and the ligation product is detected. In such an embodiment, the splint may therefore be viewed as a "splint template" or "ligation template" or "template oligonucleotide", as described herein elsewhere.

For the interaction, or more particularly ligation, to take place, one of the nucleic acids of the first and second proximity probes will typically be coupled to the antibody-binding domain by its 5'-end (leaving a free 3'-hydroxyl end), while the other domain will be coupled via its 3'-end (leaving a free 5'-phosphate end). One of the first and second proximity probes will thus be a 5' probe having a free 3'-hydroxyl group capable of interacting with the 5'-phosphate of the other 3'-probe.

To be ligatable, the respective first and second nucleic acid domains hybridize to the free splint or binding splint, respectively, with the 3'-end of one lined up to the 5'-phosphate of the other. However, as mentioned above and described in more detail below, the ligation of the respective nucleic acids need not be direct and they may be ligated together by means of an intermediary oligonucleotide, or whichever of the first or second proximity probe carries a free 3'-nucleic acid end may be extended using a polymerase to fill the gap until the first and second nucleic acids can be joined by a ligation reaction. Thus, the respective 3'- and 5'-ends need not be hybridized immediately adjacent to one another on the splint (template) but may hybridize to the splint leaving a space (or a stretch of nucleotides) between them.

The hybridization of the free splint or binding splint, respectively, simultaneously to both nucleic acids of the first and second proximity probes produces a stable duplexed structure that contains all three nucleic acids. Such a duplexed structure brings together the 3'-hydroxyl free end of the nucleic acid domain of the first proximity probe and the 5'-phosphoryl free end of the nucleic acid domain of the second proximity probe (although as mentioned above, these need not be immediately adjacently juxtaposed).

Thus, the free splint or binding splint, respectively, may include a first 3'-region of complementarity for the nucleic acid of the 5'-free proximity probe and a second 5'-region of complementarity for the nucleic acid of the 3'-free proximity probe. The first and second regions of the splint maybe 3 to 20, 6 to 17, 6 to 15 or 6 to 12 or 8 to 12 nucleotides in length, e.g. about 13 to 17, 12 to 16, 11 to 15, or 12 to 14 nucleotides in length or about 6 to 12, 7 to 11 or 8 to 10 nucleotides in length.

As described herein, amplification of the conjugation product may be used as part of the detection process. Accordingly, it may in some embodiments be desirable to design the splint so as to minimize any false amplification which may take place in such a step, for example any possibility of the splint acting as a template for the polymerase used in the amplification. Thus, for example, the splint may be provided as an RNA oligonucleotide or a DNA/RNA hybrid; Taq polymerase typically used in amplification reactions cannot use an RNA template. Alternatively, a similar effect may be achieved using a DNA splint with two short hybridization regions; since the hybridization is weak, such a splint will not template DNA polymerization at the high temperatures used in PCR.

As described herein, in one embodiment the nucleic acids of the first and second proximity probes may hybridize to the splint not immediately adjacent to each other, but to leave a gap between them. To enable their conjugation (*e.g*., ligation) a further oligonucleotide, referred to herein as a "cassette oligonucleotide", may hybridize to the splint in this gap, more particularly to span this gap. Such a cassette oligonucleotide may be hybridized with each of its ends directly adjacent to the end of each of the respective nucleic acids, such that each such nucleic acid end may be conjugated to the cassette oligonucleotide to form a single new nucleic acid product. This requires two conjugation events, both of which are templated by the splint. Both the 5'- and the 3'-end of the cassette oligonucleotide are joined (*e.g.*, ligated) to the free end of the nucleic acid of the first and second probe, as appropriate. The first and second nucleic acids are thus connected, or joined, via the cassette oligonucleotide. Such an arrangement may add flexibility to the nucleic acids of the probes. The length of the cassette oligonucleotide (and hence the gap between the ends of the first and second nucleic acids when hybridized to the splint) may vary, for example between 4 to 50, e.g., 6-30, 6-25, 6-22, 8-22, 10-22, 6-20, 8-20, 10-20 nucleotides.

The cassette oligonucleotide, which functions as an intermediary oligonucleotide in the conjugation of the first and second nucleic acids, may be added after the proximity probes have been contacted with the sample. Alternatively, it may be added at the same time or it could be pre-hybridized to the third proximity probe.

The gap may also be filled by extending the nucleic acid domain of whichever of the first or second proximity probe carries a free 3'-end, using a polymerase. Once the gap has been filled, the ends are joined by a ligation step.

In various embodiments, the sample is first contacted with at least one set of probes.

In various embodiments a sample may be assayed for two or more different target proteins. In such embodiments, the sample is contacted with a set of proximity probes for each target protein, such that the number of sets contacted with the sample may be two or more, e.g., three or more, four or more etc. Such methods find particular use in multiplex and high-throughput applications.

The amount of proximity probes that is added to a sample may be selected to provide a sufficiently low concentration of proximity probe in the reaction mixture to ensure that the proximity probes will not randomly come into close proximity with one another in the absence of binding to the respective antibodies, at least not to any great or substantial degree. As such, it is intended that only when the proximity probes bind the respective antibody through the binding interaction between the antibody-binding domains of the proximity probes and the binding sites of the antibody, do the proximity probes come into close proximity to one another. In representative embodiments, the concentration of the proximity probes in the reaction mixture following combination with the sample ranges from about 1 fM to 1 µM, such as from about 1 pM to about 1 nM, including from about 1 pM to about 100 nM.

Following combination of the sample and set(s) of proximity probes, the reaction mixture may be incubated for a period of time sufficient for the proximity probes to bind to the respective antibodies in the sample. In various embodiments, the product mixture may be incubated for a period of time ranging from about 5 minutes to about 48 hours, including from about 30 minutes to about 12 hours, at a temperature ranging from about 4 to about 50°C, including from about 20 to about 37°C. Conditions under which the reaction mixture is maintained should be optimized to promote specific binding of the proximity probe to the respective antibody, while suppressing unspecific interaction. Conditions should also allow for efficient and specific hybridization between the nucleic acids as described above.

In certain embodiments, the effective volume of the incubation mixture is reduced, at least during the portion of the incubation step in which the proximity probes are binding to the respective antibody. In these embodiments, the effective volume of the incubation mixture may be reduced for a number of different reasons. In certain embodiments, the effective volume of the incubation mixture is reduced in order to allow for the use of medium and low affinity antibody-binding domains and/or increase the sensitivity of the assay. For example, in certain embodiments where the effective volume of the incubation mixture is reduced, the antibody-binding domains may be medium or low affinity binders, by which is meant that the antibody-binding domains may have a binding affinity for their respective antibody that is less than about 10⁻⁴ M, such as about 1 nM Kd.

In various embodiments, the sensitivity of the method of the invention may be increased such that the assay can detect as few as about 100 or fewer target proteins in a 1 µl sample, including as few as about 75 or fewer target proteins in a 1 µl sample, including as few as about 50 or fewer target proteins in a 1 µl sample.

In certain embodiments, a "crowding agent" or "volume excluder" is included in the mixture during the incubation step reviewed above, *e.g*. to reduce the effective volume of the incubation mixture during binding of the proximity probes to their respective antibodies. Typically, the "crowding agent" is a water soluble macromolecular material. Suitable macromolecular materials broadly comprise biocompatible natural or synthetic polymers having an average molecular weight of from about 1500 to several million, which do not specifically interact with the other reagents in the mixture, or the product. Such polymers are known in the art as "volume-excluders", as their primary function is to occupy volume in the in vitro reaction medium and provide a highly concentrated environment for biochemical reactions, *e.g*. approximating in vivo conditions. The volume-excluding polymers must of course be sufficiently soluble to provide the required concentration. Suitable exemplary polymers include, but are not limited to: commercially available polyethylene glycol (PEG) polymers, *e.g*. having an average molecular weight greater than about 2000, FICOLL polymers such as those having an average molecular weight of about 70,000, bovine plasma albumin, glycogen, polyvinylpyrrolidone, dextran, etc. PEG polymers of higher molecular weights, especially, PEG 1450, PEG 3350, PEG 6000 (also sold as PEG 8000), and PEG 20,000, having average molecular weights of about 1450, 3000-3700, 6000-7500, and 15,000-20,000, respectively, are employed in representative embodiments. PEG 6000 and PEG 8000 are employed in representative embodiments. The concentration of the volume-excluding polymers in the incubation reaction in representative embodiments falls within a range of about 5% w/v to about 45% w/v, depending upon the type of polymer and its molecular weight. In general, it is expected that a given type of polymer of higher molecular weight need be present in lower concentration than the same type of polymer of lower molecular weight to achieve the same effect on enzyme activity.

In those embodiments where a volume excluder is employed, prior to the next step of the method, the incubation mixture may be diluted to account for the presence of the volume excluder, *e.g*. by at least about 2-fold or more, such as at least about 5-fold or more, including at least about 10-fold or more, depending on the amount of volume excluder that is present, the nature of the dilution fluid, etc. In representative embodiments the dilution fluid is water or some other suitable aqueous fluid of water and one or more solutes, *e.g*. salts, buffering agents, etc.

Instead of, or in addition to, the use of a volume excluder, the incubation mixture may be reduced in volume during incubation by removing a portion of the water from the incubation mixture, e.g., via evaporation. In these embodiments, the volume of the fluid may be reduced by at least about 2-fold or more, such as at least about 5-fold or more, including at least about 10-fold or more, as desired. Importantly, not all of the water is removed from the incubation mixture in these embodiments. Any convenient protocol may be employed for reducing the volume of the incubation mixture by removing a select portion of the water therefrom. An instrument for controlling evaporation rate by monitoring and adjusting humidity and temperature may be employed, where in certain embodiments the volume of the incubation mixture is monitored, *e.g.* by continuously measuring the volume of the incubation mixture, where when appropriately evaporated, the ligation and PCR- mixes may be added, as described above. As desired, a heating block could be used to enhance the evaporation. Alternatively, the volume of the incubation mixture may be reduced by filtrating out water. In various embodiments, a size exclusion filter is used to selectively contain molecules of sizes larger than a cut off limit while smaller molecules and water is removed by passage through the filter. The force placed on the solution to move it through the filter may be by either centrifugation or vacuum suction.

Upon binding of the binding domains of the proximity probes to the respective antibodies, the nucleic acids of the proximity probes come into close proximity to one another. As a result, the nucleic acid (free- or binding-) splint is able to bind (hybridize) to the nucleic acid of the first and second proximity probes.

Following the combination of the sample with the proximity probes, the cassette oligonucleotide may be added, if used, and allowed to hybridize, as described herein above. The nucleic acids of the first and second proximity probes (hybridized to the free- or binding-splint) are then allowed to conjugate, or interact. The reaction mixture is then assayed for the presence of any splint-mediated interaction. Thus, conjugation of the first and second nucleic acids is detected, generally by detecting the conjugation product thereof.

In general, any convenient protocol that is capable of detecting the presence of proximity dependent interactions may be employed. The detection protocol may or may not require a separation step.

In one various embodiments, the (free- or binding-) splint-mediated interaction of the first and second proximity probes (*e.g*., conjugation) is achieved by nucleic acid ligation of the free 3'-hydroxyl and 5'-phosphate ends of the nucleic acids of the first and second proximity probes, and this interaction is detected by subsequent detection of the ligated product. In these embodiments, ligation of the splint-stabilized nucleic acids of the first and second proximity probes is achieved by contacting the reaction mixture with a nucleic acid ligating activity, *e.g*. provided by a suitable nucleic acid ligase, and maintaining the mixture under conditions sufficient for ligation of the nucleic acids to occur.

As is known in the art, ligases catalyze the formation of a phosphodiester bond between juxtaposed 3'-hydroxyl and 5'-phosphate termini of two immediately adjacent nucleic acids when they are annealed or hybridized to a third nucleic acid sequence to which they are complementary (*i.e*., a template). Any convenient ligase may be employed, where representative ligases of interest include, but are not limited to: temperature-sensitive and thermostable ligases. Temperature sensitive ligases include, but are not limited to, bacteriophage T4 DNA ligase, bacteriophage T7 ligase, and *E*. *coli* ligase. Thermostable ligases include, but are not limited to, Taq ligase, Tth ligase, and Pfu ligase. Thermostable ligase may be obtained from thermophilic or hyperthermophilic organisms, including but not limited to, prokaryotic, eukaryotic, or archael organisms. Certain RNA ligases may also be employed in the methods of the invention.

In this ligation step, a suitable ligase and any reagents that are necessary and/or desirable are combined with the reaction mixture and maintained under conditions sufficient for ligation of the hybridized ligation oligonucleotides to occur. Ligation reaction conditions are well known to those of skill in the art. During ligation, the reaction mixture in certain embodiments maybe maintained at a temperature ranging from about 4°C to about 50°C, such as from about 20°C to about 37°C for a period of time ranging from about 5 seconds to about 16 hours, such as from about 1 minute to about 1 hour. In yet other embodiments, the reaction mixture may be maintained at a temperature ranging from about 35°C to about 45°C, such as from about 37°C to about 42°C, e.g., at or at about 38°C, 39°C, 40°C or 41 °C, for a period of time ranging from about 5 seconds to about 16 hours, such as from about 1 minute to about 1 hour, including from about 2 minutes to about 8 hours. In a representative embodiment, the ligation reaction mixture includes 50 mM Tris pH 7.5, 10 mM MgCl₂, 10 mM DTT, 1 mM ATP, 25 mg/ml BSA, 0.25 units/ml RNase inhibitor, and T4 DNA ligase at 0.125 units/ml. In yet another representative embodiment, 2.125 mM magnesium ion, 0.2 units/ml RNase inhibitor; and 0.125 units/ml DNA ligase are employed.

Following ligation, the ligation products (ligated nucleic acids of the first and second proximity probes) are detected as an indication of the presence, or as a measure of the amount and optionally the location, of target protein in the sample. In these embodiments, the ligated product comprises a single stranded nucleic acid molecule (which is the product of the ligation of the two proximal nucleic acids of the first and second proximity probes, and any intermediary cassette oligonucleotide, if used) terminating at each end in an antibody binding domain.

The next step of the method following the conjugation (*e.g*. ligation) step is to determine the presence of the conjugated (*e.g*. ligated) product in the reaction mixture in order to detect the target protein in the sample. In other words, the reaction mixture is screened etc. (*i.e*., assayed, assessed, evaluated, tested, etc.) for the presence of any resultant conjugation (ligation) products in order to detect the presence of target protein(s) in the sample being assayed.

The conjugated (ligated) product produced by the above-described methods may, in the broadest sense, be detected using any convenient protocol. The particular detection protocol may vary depending on the sensitivity desired and the application in which the method is being practiced. In certain embodiments, the nucleic acid ligation product may be directly detected without any amplification, while in other embodiments the detection protocol may include an amplification component, in which the copy number of the ligated product nucleic acid is increased, *e.g*., to enhance sensitivity of the particular assay. Where detection without amplification is practicable, the nucleic acid ligation product may be detected in a number of different ways. For example, one or more of the nucleic acids of the proximity probes may be directly labeled, e.g., fluorescently, or otherwise spectrophotometrically, or radioisotopically labeled or with any signal-giving label, such that the ligation product is directly labeled. In these embodiments, the directly labeled ligation product may be size separated from the remainder of the reaction mixture, including unligated directly labeled ligation oligonucleotides (*i.e*., nucleic acid oligonucleotides or cassette oligonucleotides), in order to detect the ligated nucleic acid. Alternatively, conformationally selective probes, *e.g*. molecular beacons (as described in greater detail below) may be employed to detect to the presence of the ligation product, where these probes are directed to a sequence that spans the ligated nucleic acids and therefore only exists in its entirety in the ligation product.

As indicated above, in certain embodiments of the subject methods, the detection step includes an amplification step, where the copy number of ligated nucleic acids is increased, *e.g*., in order to enhance sensitivity of the assay. The amplification may be linear or exponential, as desired, where representative amplification protocols of interest include, but are not limited to: polymerase chain reaction (PCR); isothermal amplification, etc.

Where the detection step includes an amplification step (more specifically a step of in vitro amplification of the conjugated product), the amplified product (or amplification product) may be detected, to detect target protein(s).

In various embodiments, a signal producing system that is specific for the amplification product, as opposed to double stranded molecules in general, may be employed to detect the amplification. In these embodiments, the signal producing system may include a probe nucleic acid that specifically binds to a sequence found in the amplification product, where the probe nucleic acid may be labeled with a directly or indirectly detectable label. A directly detectable label is one that can be directly detected without the use of additional reagents, while an indirectly detectable label is one that is detectable by employing one or more additional reagents, e.g., where the label is a member of a signal producing system made up of two or more components. In many embodiments, the label is a directly detectable label, where directly detectable labels of interest include, but are not limited to: fluorescent labels, radioisotopic labels, chemiluminescent labels, and the like. In many embodiments, the label is a fluorescent label, where the labeling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labeled probe nucleic acids include, but are not limited to: fluorescein, the cyanine dyes, such as Cy3, Cy5, Alexa 555, Bodipy 630/650, and the like. Other labels, such as those described above, may also be employed as are known in the art.

The next step in the subject methods is signal detection from the labeled amplification products of interest, where signal detection may vary depending on the particular signal producing system employed.

In the present invention, the level of DNA damage is determined either qualitatively or quantitatively. In various embodiments, the interaction between the nucleic acids in accordance with the present invention generates a detectable signal. Preferably, the detectable signal is fluorescence, bioluminescence or radiation. More preferably, the detectable signal is linearly related to the radiation dose to which the subject according to the present invention was subjected, or to which the cells of the subject according to the present invention were subjected. Here, the linearity is given preferably within a radiation dose range from about 0.1 Gray (Gy) to about 6.0 Gray (Gy), preferably from about 0.2 Gy to about 2.0 Gy.

In various embodiments, merely the presence or absence of detectable signal, e.g., fluorescence, is determined and used in the subject assays, e.g., to determine or identify the presence or absence of the target nucleic acid via detection of the pseudo-target nucleic acid and/or amplification products thereof. Depending on the particular label employed, detection of a signal may indicate the presence or absence of the target nucleic acid.

In those embodiments where the signal producing system is a fluorescent signal producing system, signal detection typically includes detecting a change in a fluorescent signal from the reaction mixture to obtain an assay result. In other words, any modulation in the fluorescent signal generated by the reaction mixture is assessed. The change may be an increase or decrease in fluorescence, depending on the nature of the label employed, but in certain embodiments is an increase in fluorescence. The sample may be screened for an increase in fluorescence using any convenient means, e.g., a suitable fluorimeter, such as a thermostable-cuvette or plate-reader fluorimeter. Fluorescence is suitably monitored using a known fluorimeter. The signals from these devices, for instance in the form of photo-multiplier voltages, are sent to a data processor board and converted into a spectrum associated with each sample tube. Multiple tubes, for example 96 tubes, can be assessed at the same time. Where the detection protocol is a real time protocol, e.g., as employed in real time PCR reaction protocols, data may be collected in this way at frequent intervals, for example once every 3 minutes, throughout the reaction. By monitoring the fluorescence of the reactive molecule from the sample during each cycle, the progress of the amplification reaction can be monitored in various ways. For example, the data provided by melting peaks can be analyzed, for example by calculating the area under the melting peaks and these data plotted against the number of cycles.

The spectra generated in this way can be resolved, for example, using "fits" of pre-selected fluorescent moieties such as dyes, to form peaks representative of each signaling moiety (*i.e.*, fluorophore). The areas under the peaks can be determined which represents the intensity value for each signal, and if required, expressed as quotients of each other. The differential of signal intensities and/or ratios will allow changes in labeled probes to be recorded through the reaction or at different reaction conditions, such as temperatures. The changes are related to the binding phenomenon between the oligonucleotide probe and the target sequence or degradation of the oligonucleotide probe bound to the target sequence. The integral of the area under the differential peaks will allow intensity values for the label effects to be calculated.

Screening the mixture for a change in fluorescence provides one or more assay results, depending on whether the sample is screened once at the end of the primer extension reaction, or multiple times, e.g., after each cycle, of an amplification reaction (e.g., as is done in real time PCR monitoring).

The data generated as described above can be interpreted in various ways. In its simplest form, an increase or decrease in fluorescence from the sample in the course of or at the end of the amplification reaction is indicative of an increase in the amount of target protein(s) present in the sample, *e.g*., as correlated to the amount of amplification product detected in the reaction mixture, suggestive of the fact that the amplification reaction has proceeded and therefore target protein(s) was in fact present in the initial sample. Quantification is also possible by monitoring the amplification reaction throughout the amplification process. Quantification may also include assaying for one or more nucleic acid controls in the reaction mixture, as described above.

In this manner, a reaction mixture may readily be screened (or assessed or assayed etc.) for the presence of target protein(s). The methods are suitable for detection of a single target protein as well as multiplex analyses, in which two or more different target proteins are assayed in the sample. In these latter multiplex situations, the number of different sets of probes that maybe employed typically ranges from about 2 to about 20 or higher, e.g., as up to 100 or higher, 1000 or higher, etc.

The analysis of many analytes simultaneously and in a single reaction using several different proximity ligation probe sets (multiplexing) is made possible by the increased specificity and sensitivity obtained with the binding splint method. Each probe set can be designed to produce a unique ligation product that can be used to determine the presence or absence, quantity and/or location of the analytes being interrogated by the probe set. The ligation product may be detected directly or after amplification using any of the well established methods for analysis of nucleic acid molecules known from the literature including liquid chromatography, electrophoresis, mass spectrometry, microscopy, real-time PCR, fluorescent probes etc. Of particular interest is the combination of the binding splint method with a "DNA array" read-out format. Several unique ligation products from a multiplexed proximity ligation assay may be hybridized to a standardized DNA array carrying a number of oligonucleotide sequences (tags) complementary to the ligation product sequences. Each ligation product hybridized to the array may be identified by its location on the DNA array and the detected intensity in a given hybridization spot will be indicative of the quantity of that specific ligation product and hence also of target protein(s) giving rise to that ligation product. Detection of the ligation products may be accomplished by spectrometry, fluorescence, radioisotopes etc. Fluorescent moieties may conveniently be introduced into the ligation products using fluorescently labeled primers or fluorescently labeled nucleotides in the amplification reaction (PCR). The DNA array may be a simple dot-blot array on a membrane containing a small number of spots or a high density array carrying hundreds of thousands of spots.

The method of the present invention may be modified in order to reduce the background associated with non-specific nucleic acid hybridization events. Such modifications include adjustments to the method that will reduce any non-specific nucleic acid hybridization events. In some embodiments, a protein may be added to the mixture containing the sample and the proximity probes in order to reduce weak and non-specific DNA hybridization events.

As explained above, in various embodiments the method of the invention is such that interaction between the nucleic acids of the first and second proximity probes (conjugation) should occur only if all three proximity probes are bound to their respective target protein-bound antibody. However, as is the case with all assays of this type, this cannot always be guaranteed and there may be some background interaction, or conjugation of the nucleic acid domains, if the three proximity probes come into proximity randomly in solution (the possibility of this is reduced by requiring the nucleic acids of all three probes to hybridize to one another by means of the splint, in order for such interaction to occur; the chances of all three domains coming into proximity randomly are reduced, compared to two-probe assays, nonetheless this may still under some circumstances occur). To reduce or minimize the possibility of background due to un-reacted (*i.e*. unbound) probes, blocking oligonucleotides may be used.

The blocking oligonucleotides bind (*i.e*., hybridize or anneal) to the free ends of the nucleic acids of the first and second proximity probes. Thus a blocking oligonucleotide may bind to the free 3'-OH end of the nucleic acid domain of a 5'-proximity probe and to the free 5'-phosphate end of the nucleic acid domain of a 3'-proximity probe. The binding of the blocking oligonucleotide may be out-competed in the presence of a high local concentration of the binding splint, such as occurs when all three proximity probes are bound on their respective target protein-bound antibodies. In this way the blocking oligonucleotide may prevent the first and second nucleic acids from hybridizing to the binding splint on the third proximity probe in the absence of antibody binding. Thus the free ends of the 5'-and 3'-probes may be prevented from interaction in the absence of binding to the antibody. When all three proximity probes are bound to their respective target protein-bound antibodies, the local concentration of the binding splint is sufficient to out-compete the blocking oligonucleotides; the first and second nucleic acids hybridize to the splint and the blocking oligonucleotides are replaced. The blocking oligonucleotides thus allow a competition-based strategy to be used to reduce background and thus increase sensitivity of the assay. The blocking oligonucleotides may range in length from about 4-100 nucleotides, e.g. 6-75 or 10-50. They may hybridize to a region at or near the free end of the nucleic acid of the first or second proximity probe ("near" meaning within 1-20 or 1-10, e.g. 1-6 nucleotides of the free 3'- or 5'-end). The region of hybridization may be 3-15 nucleotides long e.g. 3-12, 3-10, 3-8, 4-8, 3-6, or 4-6 nucleotides. The blocking oligonucleotides may conveniently be designed to have a hairpin structure such that the blocking oligonucleotide may be ligated to the end of proximity probes which have failed to hybridize to the splint.

The blocking oligonucleotides are typically used in an excess over the respective probes, e.g. an excess of 2-1000-fold, e.g. 20-500-fold, 50-300-fold, 100-500-fold, or 100-300-fold, e.g., 20-, 200- or 300-fold.

In the case of detecting target protein(s) with proximity probes of low affinity and slow binding kinetics, a pre-incubation step with the proximity probes at a sufficiently high concentration promotes binding of the proximity probes to the respective antibodies bound to the target protein(s). This pre-incubation step may be quickly diluted in a large volume of cold buffer (e.g., buffer that does not include target protein(s) or the proximity probes), and a portion of this dilution subsequently added to a ligation reaction mixture. This ligation reaction mixture may contain the cassette oligonucleotide (if used), ATP and ligase enzyme. The low temperature, e.g., ranging from about 0°C to about 20°C, including from about 4°C to about 10°C, minimizes the dissociation of existing proximity probe-antibody complexes while the vast dilution results in a decrease of the concentration of the unbound proximity probes, thereby lowering their reactivity and minimizing the background signal.

In such embodiments, the assay is performed by using a small incubation volume of from about 1 µl to about 20 µl, such as about 1 µl, or about 2 µl, or about 3 µl, or about 4 µl, or about 5 µl, or about 6 µl, of sample and proximity probes and then adding the cassette in a larger incubation volume of from about 8 µl to about 1.5 ml or more, such as from about 20 µl to about 1.3 ml, such as from about 50 µl to about 1 ml, such as from about 75 µl to about 800 µl, such as from about 100 µl to about 500 µl, such as from about 200 µl to about 300 µl. The effective concentration of the proximity probes in the final incubation volume is thus diluted, reducing the background while maintaining the signal since the binding between the probes and the respective antibodies does not have time to dissociate before the first and the second nucleic acids are ligated. This approach enables extremely high sensitivity as long as the ligation products can be concentrated from the larger volumes, such as over 100 µl or more, and then detecting the proximity dependent interaction. In such embodiments, the probe-probe interactions can be reduced by using single strand binding proteins.

Problems associated with complex samples may be addressed by diluting the complex sample prior to the analysis. This will greatly decrease the amount of target proteins that may be bound unspecifically to thereby lowering concentration of probes required. While the target protein will also be diluted, the high sensitivity of the proximity probing will provide good detection and quantification.

The binding splint method of the present invention may be employed homogeneously (*i.e.*, in solution) as described above, or alternatively heterogeneously, using a solid phase, for example, in which bound target protein becomes immobilized on a solid phase, permitting the use of washing steps. The use of solid phase assays offers advantages, particularly for the detection of difficult samples: washing steps can assist in the removal of inhibiting components, and target proteins can be enriched from an undesirably large sample volume. Higher concentrations and greater amounts of proximity probes can be used, as unbound target proteins and probes can be removed by washing. The ability to remove unbound or un-conjugated probes by washing also means that the solid phase assay tolerates lower purity proximity probes by comparison with the homogeneous assay.

Immobilization of the target protein on a solid phase may be achieved in various ways. Accordingly, several embodiments of the solid phase binding splint assay are contemplated. Thus the manner or means of immobilization and the solid support may be selected, according to choice, from any number of immobilization means and solid supports as are widely known in the art and described in the literature.

The solid support may be any of the well known supports or matrices which are currently widely used or proposed for immobilization, separation etc. These may take the form of particles (*e.g.*, beads which may be magnetic or non-magnetic), sheets, gels, filters, membranes, fibres, capillaries, or microtitre strips, tubes, plates or wells etc.

The support may be made of glass, silica, latex or a polymeric material. Suitable are materials presenting a high surface area for binding of target proteins according to the present invention. Such supports may have an irregular surface and may be for example porous or particulate, *e.g.* particles, fibres, webs, sinters or sieves. Particulate materials, *e.g*. beads, are useful due to their greater binding capacity, particularly polymeric beads.

Conveniently, a particulate solid support used according to the invention will comprise spherical beads. The size of the beads is not critical, but they may for example be of the order of diameter of at least 1 and preferably at least 2 µm, and have a maximum diameter of preferably not more than 10, and e.g. not more than 6 µm.

Mono-disperse particles, that is those which are substantially uniform in size (e.g. size having a diameter standard deviation of less than 5%) have the advantage that they provide very uniform reproducibility of reaction. Representative mono-disperse polymer particles may be produced by the technique described in US- A-4336173.

However, to aid manipulation and separation, magnetic beads are advantageous. The term "magnetic" as used herein means that the support is capable of having a magnetic moment imparted to it when placed in a magnetic field, and thus is displaceable under the action of that field. In other words, a support comprising magnetic particles may readily be removed by magnetic aggregation, which provides a quick, simple and efficient way of separating the particles following the target protein binding steps.

In another embodiment, an immobilized (or immobilizable) analyte-specific probe comprising only a binding domain (*i.e*., an target protein capture probe) can be used in addition to the three non-immobilized proximity probes of the homogeneous binding splint assay. Thus in such an embodiment the target protein is first captured by the immobilized or immobilizable capture probe which serves only to immobilize target protein(s) on the solid phase, and subsequently immobilized target protein(s) is/are incubated with the two or three proximity probes. In such an embodiment, the capture probe may be any binding partner capable of binding target protein(s) according to the present invention. More particularly, such a capture probe binds specifically to target protein(s) according to the present invention. Since this embodiment of the method requires the simultaneous binding of three or four probes (binding domains) to target protein(s) or target protein(s) complex, potentially three or four different epitopes can be interrogated, conferring high specificity on the assay.

In further embodiments, target protein(s) itself may be immobilized (or immobilizable) on the solid phase by, e.g., non-specific absorption. In a particular such embodiment, target protein(s) may be present within cells, being optionally fixed and/or permeabilized, which are (capable of being) attached to a solid support.

The above-described methods result in detection of free- or binding-splint-mediated proximity dependent interactions that are present in the reaction mixture, which in turn provides a measure of the amount of target protein(s) in the sample being assayed. The measure may be qualitative or quantitative.

Accordingly, the above described methods of detecting the presence of one or more target proteins in a complex sample finds use in a variety of different applications. The subject methods may be used to screen a sample for the presence or absence of one or more target proteins in a sample. As indicated above, the invention provides methods of detecting the presence or quantifying the amount of one or more target proteins in a sample.

The subject methods can be employed to detect the presence of one or more target proteins in a variety of different types of samples, including complex samples having large amounts of non-target entities, where the subject methods provide for detection of the target protein(s) with high sensitivity. As such, the subject methods are highly sensitive methods of detecting one or more target proteins in a simple or complex sample. The sample that is assayed in the subject methods is, in many embodiments, from a physiological source, as elsewhere discussed herein.

Also provided are kits that find use in practicing the subject methods, as described above. Disclosed are kits, packages, and multi-container units containing the herein described individual components to be used in the methods of the present invention. In various embodiments, these kits include a container or formulation that contains one or more of the proximity probes described herein. Packaging materials optionally include a label or instruction indicating for what diagnostic purposes and/or in what manner the agent(s) packaged therewith can be used.

For example, in various embodiments, kits for practicing the subject methods include at least one set of proximity probes, which proximity probes each include a nucleic acid acting as a reactive functionality as described above and an antibody binding domain for an antibody, which binds to epitopes on the same or different protein(s) which is/are present in DNA repair foci according to the present invention. Kits for practicing the subject methods also include at least two antibodies which bind to epitopes on the same or different protein(s) which is/are present in DNA repair foci according to the present invention.

As indicated above, the certain protocols will employ two or more different sets of such probes for simultaneous detection of two or more target proteins in a sample, *e.g.*, in multiplex and/or high throughput formats. As such, in certain embodiments the kits will include two or more distinct sets of proximity probes. Furthermore, additional reagents that are required or desired in the protocol to be practiced with the kit components may be present, which additional reagents include, but are not limited to: a ligase, cassette oligonucleotide, blocking oligonucleotides, solid support for immobilization of probe, means for immobilization of probe, binding domain or analyte, detection means, e.g. fluorescently labeled nucleotides or oligonucleotides, pairs of supplementary nucleic acids, single strand binding proteins, and PCR amplification reagents (e.g., nucleotides, buffers, cations, etc.), and the like. In certain embodiments, the kits may include elements employed in reducing the effective volume of an incubation mixture, as reviewed above, e.g., a volume excluder. The kit components may be present in separate containers, or one or more of the components may be present in the same container, where the containers may be storage containers and/or containers that are employed during the assay for which the kit is designed.

In addition to the above components, the subject kits may further include instructions for practicing the subject methods. These instructions may be present in the subject kits in a variety of forms, one or more of which may be present in the kit. One form in which these instructions may be present is as printed information on a suitable medium or substrate, *e.g*., a piece or pieces of paper on which the information is printed, in the packaging of the kit, in a package insert, etc. Yet another means would be a computer readable medium, *e.g*., diskette, CD, etc., on which the information has been recorded. Yet another means that maybe present is a website address which may be used via the internet to access the information at a removed site. Any convenient means may be present in the kits.

Accordingly, in a further aspect the present invention provides a kit for use in a method for determining the level of DNA damage in cells of a subject subjected to ionizing radiation in accordance with the present invention, said kit comprising at least one set of at least first and second proximity probes each comprising a nucleic acid acting as a reactive functionality and a binding moiety with affinity for at least two antibodies which bind to epitopes on the same or different protein(s) which is/are present in DNA repair foci according to the present invention.

As indicated above, the means for conjugation may be a ligase enzyme, and such means may optionally further comprise the reagent necessary for the ligase reaction (*e.g.* nucleotides etc). The means for detecting the conjugation, may be any of the means discussed above in the context of the assay methods, *e.g*. a label provided on the nucleic acid domains of the first and second probe or it may be amplification means and means for detecting amplification products thereof e.g. reagents for a PCR reaction (*e.g.*, amplification primers, and optionally polymerase and/or nucleotides, etc.) and for detecting PCR amplicons etc. (e.g., Taqman® probes etc.).

The kit may further optionally comprise a cassette oligonucleotide and/or blocking oligonucleotides for the first and second proximity probes.

The kit may further optionally comprise an immobilized capture probe for target protein(s), or a capture probe provided with means for immobilization. Alternatively, the kit may comprise a solid phase for capture of, or binding to, target protein according to the present invention, or one or more said first, second or third proximity probes may be immobilized or provided with means for immobilization.

In a further aspect, the present invention provides a device suitable for determining the level of DNA damage in cells of a subject subjected to ionizing radiation in accordance with the means and methods of the present invention. Said device is shaped in a way such that it allows the performance of the method of the present invention. For example, said device comprises one or more containers comprising one or more antibodies which bind to epitopes on the same or different protein(s) which is/are present in DNA repair foci. Similarly, said device comprises one or more containers comprising one or more proximity probes as described herein. For example, said container comprises a first and/or second proximity probes each comprising a binding moiety with affinity for said antibodies and a nucleic acid acting as a reactive functionality. It is envisaged that said one or more antibodies and/or proximity probes are immobilized, e.g., on beads such as micro-or nanobeads, a plastic surface, glass surface, and the like.

Said device may also comprise pumps, valves, tubes and the like to, e.g. allow pumping of liquids from one or more of said containers into, e.g. a reaction vessel or chamber.

In various embodiments, the subject that is subjected to a detection method according to the present invention is a subject suffering from DNA damage in accordance with the present invention. Preferably, the subject was subjected to ionizing radiation prior to performing the methods of the present invention. In various embodiments, the subject is still subjected to ionizing radiation while performing the methods of the present invention. In various embodiments, the subject is assumed that it was subjected to ionizing radiation prior to performing the methods of the present invention. Preferably, the subject is a human or animal subject, more preferably a human being. Even more preferably, the human being is a patient subjected to radiotherapy during cancer treatment. Accordingly, the patient is a cancer patient. Accordingly, the methods of the present invention may be used in detecting cancer subjects which are hypersensitive to radiotherapy.

### EXAMPLES

A better understanding of the present invention and of its advantages is given from the following examples, which are offered for illustrative purposes only and which are not intended to limit the scope of the present invention in any way.

The assay was established for the detection of repair complexes in cultured cell lines and in human primary fibroblasts and lymphocytes. The assay was validated by using repair-deficient cells mutated in ATM or LIG4 as well as by using siRNA mediated knockdown of ATM. Gamma-H2AX containing protein complexes were quantified between 1 and 24 h after irradiation to monitor DNA double strand break repair kinetics. All repair-deficient cell lines display an about 4-fold increased number of residual protein complexes after 24 h repair-time, evidencing their reduced repair capacity.

So far Proximity Ligation assays (PLA) were conducted with the human retinal pigment epithelial cell line hTERT-RPE, primary human fibroblasts (1BR3, ATBR) and with primary human lymphocytes from healthy donors.

### Materials and Methods

### Cultivation and irradiation of cells

The telomerase-positive, immortalized h-TERT RPE was cultured in Dulbecco's Modified Eagle Medium (DMEM/F12) containing 2.5 mM L-glutamine, 10% heat inactivated fetal bovine serum and 0.25% sodium bicarbonate. Cells were incubated at 37°C in a humidified atmosphere containing 5% CO₂. Primary human fibroblasts 1 BR3 and ATBR1 were cultured in MEM medium supplemented with 20% FCS. Cells were incubated at 37°C in a humidified atmosphere containing 5% CO₂. All cell lines and primary cells were grown in appropriate medium in 60 mm culture dishes. Before treatment RPE cells and primary fibroblasts were trypsinized and cells were seeded on 18-well slides (IBIDI, Germany, µ-slides, 18 well flat) (1000 cells/well). 24 h later exponentially growing cells were irradiated.

For the detection of protein interactions in primary lymphocytes 8 ml blood were collected from donors in heparinized tubes and. peripheral blood mononuclear cells (PBMC) were isolated by the FICOLL method. Therefore, blood was diluted 1:1 in HBSS (Hank's Buffered Salt Solution), layered on the FICOLL solution and centrifuged for 40 min at 1200 x g. Serum was discarded and the PBMC containing layer was transferred to a 12 ml reaction tube. After washing the cells with HBSS the PMBCs were stored at -80°C. For the PLA assay PBMCs were cultivated for 24 h in RPMI medium containing 20 % FCS at 37°C and irradiated in culture flasks.

All irradiations were carried out with a cesium-137 source at a dose rate of 0.53 Gy/min with the indicated doses.

### Fixation and primary antibody incubation

Fixation of all cells was carried out after 90 min incubation at 37°C post irradiation. Primary lymphocytes were fixed in 3 % paraformaldehyd for 30 min on ice. Afterwards cells were washed twice with PBS (137 mM NaCl, 2.7 mM KCI, 10 mM sodium phosphate dibasic, 2 mM potassium phosphate monobasic and a pH of 7.4), resuspended in methanol, transferred to the IBIDI slides and the methanol was evaporated. Adherent cell lines and primary fibroblasts were fixed in 4 % paraformaldehyd for 15 min at room temperature. For permeabilization cells were incubated for 10 min in PBS supplemented with 0.2 % Triton at room temperature. After washing the slides with PBS, cells were blocked with PBS⁺ (1 g BSA + 0.15 g glycin in 100 ml PBS) for 1 h at room temperature. After blocking 30 µl of a mixture of the two primary antibodies (diluted 1:200 in PBS⁺) were added to each well and the cells were incubated at 4°C for at least 3 h. To detect the interaction between phospho-H2AX and 53BP1 the antibodies anti-53BP1 (host: rabbit, NB100-304, NOVUS BIOLOGICAL) and anti-phospho-H2AX (Ser139) (host: mouse, clone JBW301, UPSTATE/Chemicon) were used.

### Proximity ligation procedure

PLA probes directed against the constant domains of the primary antibodies were diluted by a factor of 1:5 in antibody dilution solution and 30 µl of the solution were added to each well. Now slides were incubated in a pre-heated humidity chamber for 2 h at 37°C. PLA probes were tapped off and slides were washed (2 x 5 min) using TBS-T (20 mM Tris, 137 mM NaCl, 0.1 % Tween 20, pH 7,6).

Hybridization stock solution was diluted 1:5 in high purity water and was added to each sample. After 30 min at 37°C in the pre-heated humidity chamber the hybridization solution was removed and the slides were washed with TBS-T for 1 min under gentle agitation. Subsequently, Duolink ligation stock solution was diluted 1:5 with high purity water, Duolink ligase was added (1 µl/30 µl ligation solution) and the solution was vortexed. Afterwards 30 µl were added to each well and slides were incubated for 30 min at 37°C in a humidity chamber.

The ligase solution was removed and 30 µl of the Duolink Amplification solution (1:40 diluted amplification stock solution) containing 0,5 µl polymerase per 30 µl solution were added to each well, after 2 x 2 min washing with TBS-T. The slides were incubated for 90 min in a humidity chamber at 37°C.

For the detection of amplification products all following steps were conducted in the dark. First, the detection stock was diluted 1:5 in high purity water, the amplification solution was removed and slides were washed 2 x 2 min with TBS-T under gentle agitation. Then 30 µl of the detection solution were added to each well and the slides were incubated for 60 min at 37°C in a humidity chamber. For the final washing slides were successively washed for 2 min with 2 x SSC (0.03 M trisodium citrate, 0.3 M sodium chloride, pH 7.0), 1 x SSC, 0.2 x SSC, 0.1 x SSC, 0.05 x SSC and 0.02 x SSC. Finally, slides were covered with 70 % ethanol for 1 min and dried afterwards. To prepare for imaging 30 µl of Duolink mounting medium were applied to each sample.

### Imaging

Slides were analyzed in a fluorescence microscope immediately after preparation. The fluorophores were excitated at 543 nm and the emission wavelength was 563 nm. The detection stock also contains Hoechst 33342 for nuclear staining.

### Illustrative Examples

### Dose-dependent increase of PLA signals

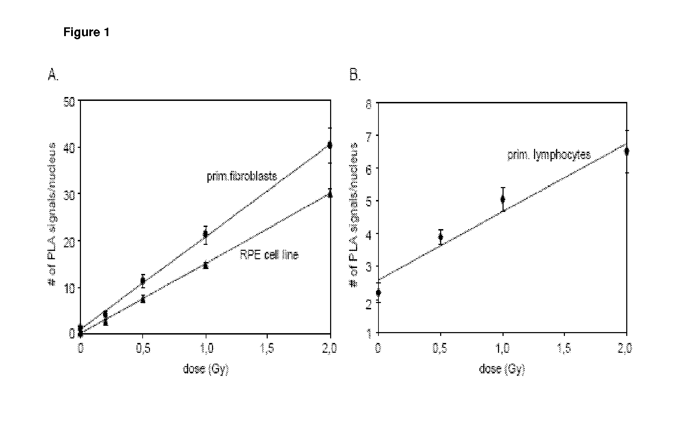

The assay was established for the detection of repair complexes in a cultured human retinal epithelial cell line (hTERT-RPE), in human primary fibroblasts and in human primary lymphocytes. For this approach the formation of the DNA damage signalling complex between the proteins γH2AX and 53BP1 was detected by the PLA assay using anti-γH2AX and anti-53BP1 antibodies. Therefore RPE cells or primary human fibroblast in logarithmic growth phase were irradiated with 0; 0.2; 0.5; 1.0 and 2.0 Gy. After a post irradiation incubation of 1h at 37 °C the PLA protocol was performed as described. For the determination of PLA signals per dose the PLA signals from at least 150 nuclei were counted and the means of four independent experiments were shown. For both cell types we detected a linear increase in the number of PLA signals. In non-irradiated RPE cells we detected around 0.8 signals per nucleus, resulting from DNA double strand breaks occurring in S-phase. After irradiation the number of PLA signals per nucleus constantly increases with the radiation dose. 0.2 Gy resulted in 2,6 signals per nucleus, after 0.5 Gy 8,2 signal per nucleus and after 1 Gy 14,9 signals were detected, while the highest investigated dose of 2.0 Gy yielded 30,2 signals per nucleus (Fig 1A). For the primary fibroblasts 1,2 signals per nucleus were detected in non-irradiated cells. 0,2 Gy resulted in 4,3 signals, 0,5 Gy in 11,4 signals, 1 Gy in 21,3 signals and 2 Gy in 40,4 signals per nucleus (Fig.1A).

For the detection of DNA double strand breaks in primary lymphocytes, whole blood was collected from a healthy donor and in vitro irradiated with 0; 0.5; 1.0 and 2.0 Gy. After 1h incubation at 37°C mononuclear cells were isolated and the PLA protocol was carried out. In this experiment one antibody recognized endogenous non-phosphorylated H2AX and the second antibody was directed against phosphorylated H2AX (γ-H2AX). In non-irradiated cells we found about 2.3 PLA signals per nucleus. After irradiation we also detected a dose dependent increase in nuclear PLA signals, evidenced by 3.8 (0.5 Gy); 5.1 (1 Gy) and 6.4 (2 Gy) PLA signals per nucleus (Fig1 B).

### Validation of the PLA assay using repair-deficient cells

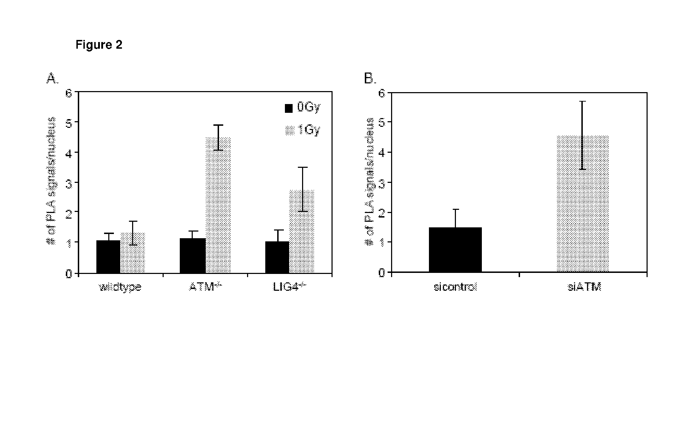

For the validation of the assay we used repair-deficient primary fibroblasts mutated in the DNA double strand recognition and repair proteins ATM or LIG4. Furthermore we compared control siRNA transfected RPE cells with siATM transfected cells, which display a specific knockdown (reduction) of the ATM protein. Anti-γH2AX and anti-53BP1 were used as primary antibodies. γ-H2AX/53BP1 containing protein complexes were quantified without irradiation and 24 h after irradiation with 1 Gy to monitor DNA double strand break repair kinetics. All repair-deficient cell lines displayed an about 4-fold increased number of residual protein complexes after 24 h repair-time (Fig. 2.). Functionally this result confirmed their reduced repair capacity. Furthermore it emphasized the suitability of the PLA assay as a sensitive tool to measure DNA double strand breaks.

### REFERENCES

[1] Rodrigue A, Lafrance M, Gauthier MC, McDonald D, Hendzel M, West SC, Jasin M, Masson JY, Interplay between human DNA repair proteins at a unique double-strand break in vivo. EMBO J. 2006 Jan 11;25(1):222-31.
[2] Rogakou EP, Pilch DR, Orr AH, Ivanova VS, Bonner WM., DNA double-stranded breaks induce histone H2AX phosphorylation on serine 139., J Biol Chem. 1998 Mar 6;273(10):5858-68.
[3] Rogakou EP, Boon C, Redon C, Bonner WM., Megabase chromatin domains involved in DNA double-strand breaks in vivo. J Cell Biol. 1999 Sep 6;146(5):905-16.
[4] Wang H, Wang M, Wang H, Böcker W, Iliakis G., Complex H2AX phosphorylation patterns by multiple kinases including ATM and DNA-PK in human cells exposed to ionizing radiation and treated with kinase inhibitors. J Cell Physiol. 2005 Feb;202(2):492-502.
[5] Furuta T, Takemura H, Liao ZY, Aune GJ, Redon C, Sedelnikova OA, Pilch DR, Rogakou EP, Celeste A, Chen HT, Nussenzweig A, Aladjem MI, Bonner WM, Pommier Y., Phosphorylation of histone H2AX and activation of Mre11, Rad50, and Nbs1 in response to replication-dependent DNA double-strand breaks induced by mammalian DNA topoisomerase I cleavage complexes. J Biol Chem. 2003 May 30;278(22):20303-12.
[6] Rothkamm K, Löbrich M., Evidence for a lack of DNA double-strand break repair in human cells exposed to very low x-ray doses. Proc Natl Acad Sci U S A. 2003 Apr 29;100(9):5057-62.
[7] Ohnishi T, Mori E, Takahashi A., DNA double-strand breaks: their production, recognition, and repair in eukaryotes. Mutat Res. 2009 Oct 2;669(1-2):8-12. Review.
[8] Söderberg O, Leuchowius KJ, Kamali-Moghaddam M, Jarvius M, Gustafsdottir S, Schallmeiner E, Gullberg M, Jarvius J, Landegren U., Proximity ligation: a specific and versatile tool for the proteomic era. Genet Eng (N Y). 2007;28:85-93. Review
[9] Gebauer M. and Skerra A. Engineered protein scaffolds as next-generation antibody therapeutics. Curr Opin Chem Biol. 2009 13(3):245-55

## Claims

1. A method for determining the level of DNA damage in cells of a subject subjected to ionizing radiation comprising
(a) contacting a sample comprising cells obtained from said subject with at least two antibodies which bind to epitopes on the same or different protein(s) which is/are present in DNA repair foci;
(b) contacting said sample with at least a first and second proximity probes each comprising a binding moiety with affinity for said antibodies and a nucleic acid acting as a reactive functionality, coupled thereto;
(c) allowing the binding moiety of said proximity probes to bind to said antibodies and allowing the nucleic acids of said proximity probes to interact with each other if the proximity probes are in close proximity to each other; and
(d) detecting the degree of interaction between the nucleic acids, thereby determining the level of DNA damage.

2. The method according to claim 1, further comprising amplifiying the interacted nucleic acids.

3. The method of claim 1 or 2, wherein determining the level of DNA damage is qualitatively and/or quantitatively.

4. The method of any one of the preceding claims, wherein the interaction between the nucleic acids in step (d) generates a detectable signal.

5. The method of claim 4, wherein said detectable signal is linearly related to the radiation dose to which cells of a subject were subjected.

6. The method of claim 5, wherein the linearity is given within a radiation dose range from about 0.1 Gy to about 6.0 Gy, preferably 0.2 Gy to about 2.0 Gy.

7. The method of any one of the preceding claims, wherein the binding moiety of the proximity probe is an antibody or aptamer.

8. The method of claim 7, wherein said antibody is directed against the Fc portion of the antibodies which bind to protein(s) which is/are present in DNA repair foci.

9. The method of any one of the preceding claims, wherein the protein(s) which is/are present in DNA repair foci is/are DNA repair protein(s).

10. The method of claim 9, wherein said DNA repair protein is selected from the group consisting of H2AX, γH2AX, 53BP1, TRF2, H2AX, ERK, RAD50, RAD51, RAD54, Ku, XRCC4, NBS1, MRE11, MDC1, ATM, Ku70, Ku80, DNA-PK, Ligase-4, XRCC4. WRN, BLM, RPA, RNF168, BRCA1, RNF8 and Rap80, preferably said DNA repair protein is γH2AX and/or 53BP1.

11. The method of any one of the preceding claims, comprising
(b') contacting in step (b) said sample with a third proximity probe which comprises a binding moiety with affinity for an antibody which binds to an epitope on the same or different protein(s) which is/are present in DNA repair foci, and a third nucleic acid acting as a reactive functionality, coupled thereto, the nucleic acid of said first proximity probe has a 3' free nucleic acid (A), the nucleic acid of said second proximity probe has a 5' free nucleic acid (B), and the nucleic acid of said third proximity probe has both 3' and 5' free nucleic acids (C), wherein the 3'-end of A interacts with the 5'-end of C and the 3'-end of C interacts with the 5'-end of B; and
(c') conjugating the nucleic acids of said first and second proximity probes.

12. The method of claim 11, wherein the nucleic acid of said third proximity probe is a splint which is capable of hybridising at least to the nucleic acid of said first and second proximity probes, wherein when all of the three proximity probes bind to said antibodies, the nucleic acid of said first and second proximity probes are directly or indirectly conjugatable by means of a ligation reaction templated by said hybridized splint of said third proximity probe.

13. The method of any one of the preceding claims, wherein at least one of said at least first, second and third proximity probes is immobilized, or is capable of being immobilized, on a solid phase.

14. Use of the method of any one of the preceding claims for
(i) detecting subjects which are hypersensitive to radiation therapy;
(ii) avoiding radiation injury to subjects before receiving further radiation doses;
(iii) stratifying subjects in need of a countermeasure against radiation injury;
(iv) fast detection of subjects exposed to radiation.

15. A kit for determining the level of DNA damage in cells of a subject subjected to ionizing radiation comprising at least one set of at least first and second proximity probes as defined in claim 1.
